(19) **Europäisches Patentamt / European Patent Office / Office européen des brevets**

(11) **EP 4 163 296 A1**

(12) **EUROPEAN PATENT APPLICATION**

(43) Date of publication:
**12.04.2023 Bulletin 2023/15**

(21) Application number: **22200939.1**

(22) Date of filing: **11.10.2022**

(51) International Patent Classification (IPC):
**C07K 14/705** (2006.01)   **A61P 27/02** (2006.01)
**A61K 38/17** (2006.01)   **A61K 48/00** (2006.01)

(52) Cooperative Patent Classification (CPC):
(C-Sets available)
**C07K 14/705; A61K 35/761; A61K 38/1709;**
**A61K 48/005; A61K 48/0058; A61K 48/0075;**
**A61P 27/02;** A01K 2217/072; A01K 2217/075;
A01K 2217/077; A01K 2227/105; A01K 2267/035;
C12N 2750/14122; C12N 2750/14143;
C12N 2750/14144;                              (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **11.10.2021 EP 21201958**

(71) Applicant: **Sparingvision**
**75008 Paris (FR)**

(72) Inventors:
• **DALKARA, Deniz**
  **94140 ALFORTVILLE (FR)**
• **SIMON, Cardillia-Joe**
  **93270 SEVRAN (FR)**

• **HERLITZE, Stephan**
  **44797 BOCHUM (DE)**
• **SAHEL, José-Alain**
  **75013 PARIS (FR)**
• **AUDO, Isabelle**
  **91400 ORSAY (FR)**
• **PICAUD, Serge**
  **77210 AVON (FR)**
• **BERTIN, Stéphane**
  **78110 LE VESINET (FR)**

(74) Representative: **Plasseraud IP**
  **66, rue de la Chaussée d'Antin**
  **75440 Paris Cedex 09 (FR)**

Remarks:
The complete document including Reference Table(s) and the Sequence Listing(s) can be downloaded from the EPO website

(54) **G-PROTEIN-GATED-K+ CHANNEL-MEDIATED ENHANCEMENTS IN LIGHT SENSITIVITY IN PATIENTS WITH ROD-CONE DYSTROPHY (RCD)**

(57)    The present invention concerns a new gene therapy approach to increase light-sensitivity in degenerating cones in advanced stages of rod-cone dystrophy (RCD) mediated by G-protein-gated-K+ channel (GIRK), in particular GIRK2, activated by G proteins recruited by cone opsin expressed in degenerating cones for use in the treatment of patients with RCD.

EP 4 163 296 A1

(52) Cooperative Patent Classification (CPC): (Cont.)
C12N 2830/008

C-Sets
**A61K 35/761, A61K 2300/00;**
**A61K 38/1709, A61K 2300/00**

**Description**

**Technical field of the invention**

The present invention concerns a new gene therapy approach to increase light-sensitivity in degenerating cones in advanced stages of rod-cone dystrophy (RCD) mediated by G-protein-gated-K+ channel (GIRK), in particular GIRK2, activated by G proteins recruited by cone opsin expressed in degenerating cones.

In the description below, references in square brackets ([ ]) refer to the list of references at the end of the text.

**State of the art**

Retina is the light sensitive tissue of the eye composed of three layers of neurons interconnected by synapses. The primary neurons of the retina are the light-sensing photoreceptors (PR), which are of two types: the rods for night vision and the cones for daylight vision. Cone-mediated vision is mostly supported by the fovea and is responsible for high acuity central vision most valuable to our daily visual tasks (Sinha et al., 2017) [1]. The light sensitive G protein coupled receptors that link photon capture to intracellular signaling leading to membrane hyperpolarization in photoreceptors are called opsins (Yau and Hardie, 2009) [2]. There is one type of rod opsin found in rods and three types of cone opsins - responsible for trichromatic vision - in the primate retina. The structural properties and phototransduction cascades are similar between these opsins.

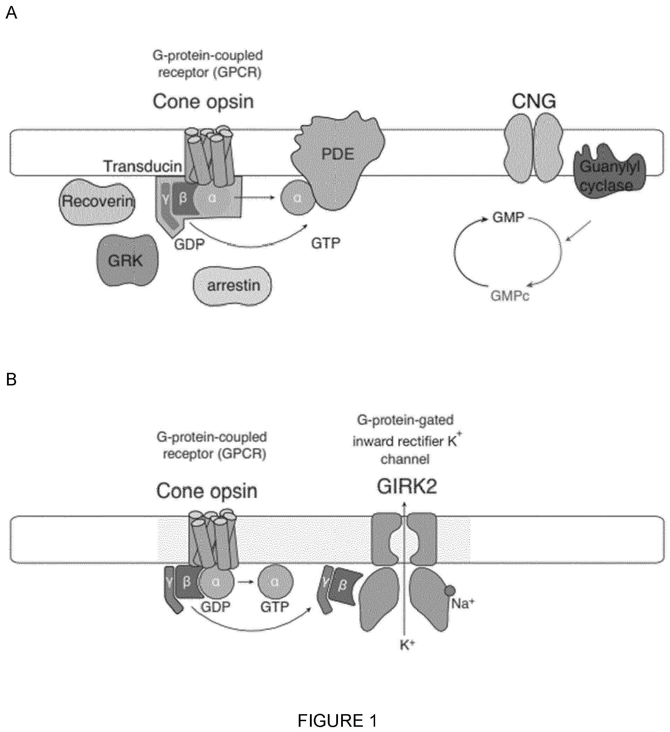

The phototransduction cascade is composed of several proteins that are concentrated in the photoreceptor outer-segments in normal retinas (Figure 1A). The role of the photoreceptor is to sense light via this phototransduction cascade and induce an electrical signal that is then processed and transmitted towards downstream neurons (Ebrey and Koutalos, 2001) [3].

The absorption of a photon activates the opsin composed of two parts: the protein part, and the light absorbing part, which is the retinal - a derivative of vitamin A. The latter isomerizes from 11-cis-retinal (dark adapted state) into all-trans-retinal configuration (light adapted state). As a result, the opsin becomes catalytically active recruiting the G protein transducin. The $\alpha$-subunit of transducin is activated by the replacement of GDP by GTP. After, the $\alpha$-subunit dissociates from the $\beta\gamma$-subunits to activate the membrane-associated phosphodiesterase 6 (PDE) by binding its two inhibitory $\gamma$ subunits. The activated PDE hydrolyses cGMP into GMP. The reduction of cGMP clones the nucleotide-gated channels (CNG) and this stops cation entry, resulting in PR hyperpolarization and reduction in glutamate release by the photoreceptor (Larhammar et al., 2009) [4].

In order to respond to another photon, this phototransduction cascade is deactivated by two mechanisms: (i) the transducin inactivates itself by hydrolyzing the bound GTP and (ii) the rhodopsin kinase (GRK) phosphorylates the opsin that interacts with the regulatory protein arrestin, leading to opsin inactivation. Retinal is then recycled by the retinal pigment epithelium (RPE) and Müller glial cells. Each and every protein of this cascade plays an important role in converting the light signal into an electrical signal conveyed to the second and third order neurons (Maeda et al., 2003) [5].

Inherited retinal degenerations are mostly due to mutations in photoreceptor or RPE cells leading to the degeneration of the rods, followed by cone outer segment degeneration eventually leading to blindness (Buch et al., 2004) [6]. Among them, rod-cone dystrophy (RCD) represents the largest category where genetic causes are highly heterogeneous. More than 60 different genes expressed in rod photoreceptors or the retinal pigment epithelium are involved (Wright et al., 2010) [7]. The first gene that has been linked to RCD is the rhodopsin gene RHO that counts for 25% of RCD autosomal dominant cases. Many other causative genes have also been identified: the cGMP-PDE subunit gene and the cyclic GMP gated channel protein $\alpha$ subunit gene. Although there are many causative genes, the resulting RCD phenotype is the same across different mutations (Ferrari et al., 2011) [8]. The common RCD phenotype is characterized by the progressive rod degeneration, causing night blindness, and followed by progressive peripheral cone degeneration, causing "tunnel vision", mediated entirely by the remaining foveal cones then eventually resulting in complete blindness in the latest stages of disease. Usually, when patients are diagnosed with RCD, they already show night blindness, meaning their rods have degenerated. However, the cones remain until the late stages of the disease; particularly in the foveal region responsible for high acuity leading to tunnel vision in early stages (Li et al., 1995) [9]. In later stages of the disease, these cones lose their outer segment structures leading to complete blindness before the complete loss of the cone soma and pedicle (Li et al., 1995) [9].

In order to preserve the vision in these patients presenting light sensitive cone cell bodies, one innovative strategy is retinal gene therapy, which broadly refers to the transfer of a therapeutic gene into retinal cells to mediate a therapeutic effect (Bennet, 2017) [10]. Although the first successful clinical trials of gene therapy have focused on gene replacement, where a gene carrying a recessive mutation is replaced by a functional cDNA copy, this strategy is limited because it cannot be used for the majority of retinal degenerations (Bennet, 2017) [10]. For example, in RCD, the huge variability of mutations makes it difficult to apply to each specific mutation. Moreover, dominant mutations cannot be treated using this approach. Furthermore, in 50% of cases, the causative mutation is not elucidated or the rod photoreceptors bearing the most frequent mutations are already lost (Dalkara et al., 2015) [11]. For these reasons, mutation-independent gene therapies that can be applied beyond the loss of rods must be developed to treat a large number of patients without knowledge of the mutant gene.

Taking this goal into account, previous studies used the ectopic expression of microbial opsins, like channelrhodopsin in bipolar cells or halorhodopsin in cones PRs, to modulate membrane potential and induce a depolarization or hyperpolarization respectively (Busskamp et al., 2012; Dalkara and Sahel, 2014; Scholl et al., 2016) [12-14]. Thus, in RCD, optogenetics can be used as a therapeutic strategy to restore vision in blind retinas (Baker and Flannery, 2008) [15]. There are many parameters to consider: (i) the choice of cell target to make the most out of the retinal circuitry sending the most interpretable signal to the brain (ii) the choice of the appropriate optogenetic tool to obtain a close to natural electrophysiological response in these target cells. Concerning the second point, the relatively weak capacity of microbial opsins has been a major limitation: potential immunogenicity of using an opsin from prokaryotic species, high intensities required due to the lack of signal amplification by these directly light gated channels and pumps originating from single cell microorganisms, as in the case of halorhodopsin (Baker and Flannery, 2008) [15], (Cehajic-Kapetanovic et al., 2015; Gaub et al., 2015; Van Gelder and Kaur, 2015; van Wyk et al., 2015) [16-19]. Unlike rhodopsins or cone opsins, microbial opsins are not able to activate G protein coupled cascades such as the phototransduction cascade present in healthy retinas. One of the possible ways to go beyond the limits of microbial opsins is the use of animal opsins, which are all G protein coupled receptors. However all work in this field has so far been focused on inner retinal neurons (Berry et al., 2019; Cehajic-Kapetanovic et al., 2015; De Silva et al., 2017; Gaub et al., 2015; Lin et al., 2008; van Wyk et al., 2015) [20, 16, 21, 17, 22, 19].

In a previous study, it has been shown that light-activation of two animal cone opsins can stimulate the $G_{i/o}$ signalling pathways in human kidney cells and in neuronal cells *in vitro* and *in vivo* (Masseck et al., 2014) [23]. This pathway is involved in fast dampening of neuronal activity and inhibition of intrinsic ion channels. However, it has also been shown that animal cone opsins can directly modulate the G protein-gated inwardly rectifying potassium channels (GIRK) upon co-expression in any given cell (Berry et al., 2019; Masseck et al., 2014) [20, 23]. GIRK channels are composed of two subunits. There are four types of subunits: GIRK1 to 4. GIRK1 and 3 cannot form homotetramers; they have to be associated with GIRK2 to be functional (Mark and Herlitze, 2000) [24]. Conversely, GIRK2 alone can form homotetramers. The GIRK channel is predominantly closed at resting membrane potentials. After its activation by the $\beta\gamma$ subunit of a $G_{i/0}$ protein, potassium ions flow out of the cell, thus, hyperpolarizing the neuron (Figure 1B). It has therefore been possible to use vertebrate cone opsins SWO (for short wavelength opsin) and LWO (for long wavelength opsin) for repetitive $G_{i/0}$ activation of a specific wavelength *in vivo* in the anxiety circuitry, and the combination of cone opsins with GIRK has proven more efficient that microbial opsins at low light intensities (Masseck et al., 2014) [23].

**Description of the invention**

The Inventors have thus investigated if it was possible to implement this cone opsin based system in a vision restoration setting and investigated patient retinas in preparation for clinical candidate selection. Therefore in order to develop a light-sensitive cone reactivation strategy, the expression of the phototransduction cascade elements in cones during degeneration was first examined in two RCD mouse models. After exploring the phosphotransduction cascade in two RCD mouse models, a target molecule approach acting via $G_{i/0}$ proteins recruited by the activation of remaining cone opsin was proposed for the first time. It has thereby created a new "short phototransduction cascade" that is independent from the expression of PDE and transducin.

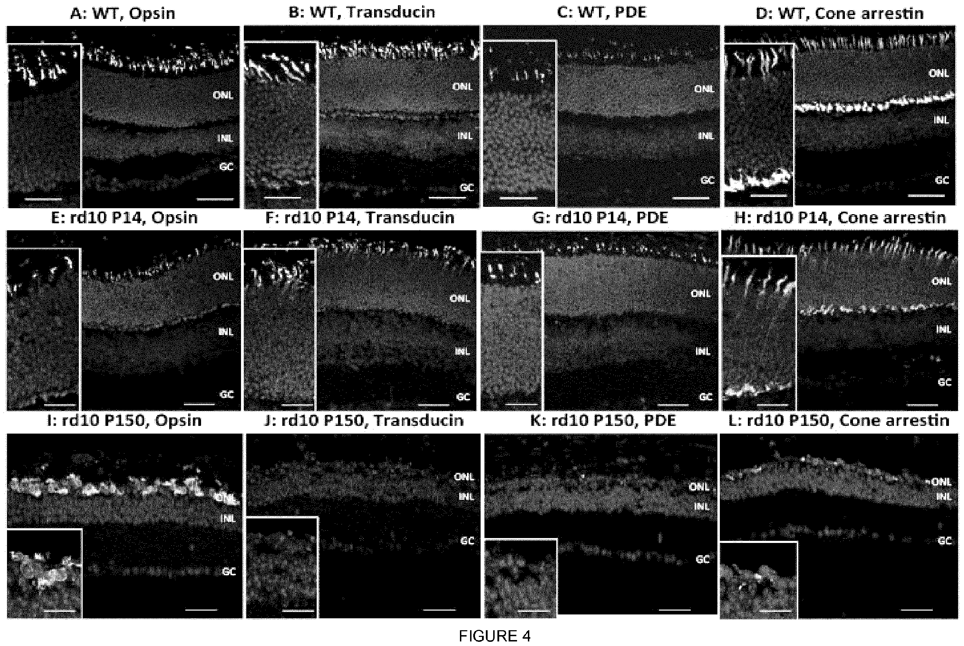

First, the state of the endogeneous phototransduction cascade in degenerating cones through the progression of disease was investigated. In both mouse models, only opsin and arrestin were found to migrate to the cone cell bodies after outer segment degeneration. Thus it was hypothesized that cone reactivation based on cone opsin signaling may be feasible, which in turn will allow to recover high sensitivity vision. It was found that endogenous cone opsins were still expressed at the level of the cone cell body in rd10 mouse model (Figure 4) suggesting the possibility of linking their activity to GIRK channels, in particular GIRK2 channel, even in absence of transducin and phosphodiesterase (Figure 1B). In this configuration, the opening of GIRK2 channel will allow the exit of potassium ions due to the resting membrane potential of dormant cones (Busskamp, 2010) [25]. $K^+$ efflux via the GIRK2 channel will hyperpolarize the cones in response to light as it was seen in the two mouse models of RCD.

Next, the target GIRK2 channel activated by G proteins recruited by cone opsin was expressed in degenerating cones. Moreover, since the remaining opsin in the cone cell bodies is still functional and sufficient to induce a light response in the degenerated cones, the insertion of GIRK2 in all cones leads to light responses following the spectral properties of each of the opsins preserving color vision. The results pointed towards enhanced light-sensitivity in cones of RCD retinas during and after degeneration of cone outer-segments. The results thus demonstrated that vertebrate light sensitive proteins combined with GIRK channel, in particular GIRK2 channel, activated by an endogenous G protein, could improve the visual function in the two mouse models, as demonstrated by electroretinography and behaviour. This is the first time that a light insensitive mammalian ion channel has been linked to intrinsic opsins providing new avenues in vision restoration that can be implemented to increase light sensitivity even before complete outer segment degeneration. Since this new system makes use of intrinsic opsins expressed in degenerating cones, it also enables for the first time, color vision restoration/maintenance.

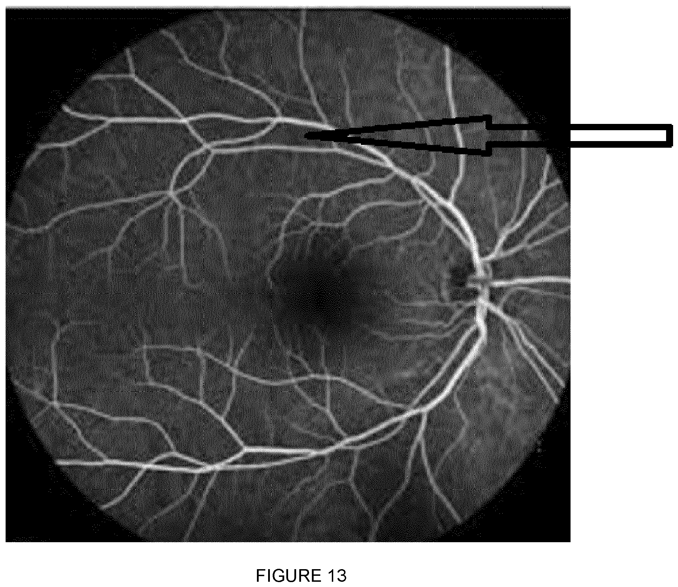

Similarly to the RCD mouse models, the cone opsin and the cone arrestin remain in the cone cell bodies of RP human patients (Figure 13). This result demonstrates the feasibility of reactivating the cone function in the foveal region of RCD human patients with the short GIRK2/opsin phototransduction cascade. The activation of the remaining cone opsin by a light stimulus will trigger the short phototransduction cascade and lead to vision restoration in RCD patients, even at an intermediate or advanced stage of the disease.

This new approach has thus the potential to maintain and/or restore, high acuity and color vision requiring only low light intensities in human patients.

A clear advantage of microbial opsins is their robustness and millisecond scale kinetics (Packer et al., 2013) [26]. For systems using other opsins, it should be considered that in order to respond to another light stimulus, the cascade has to be deactivated to recover light sensitivity. In absence of this, cones may stay hyperpolarized after GIRK2 channel activation limiting their ability to modulate synaptic transmission at a movie rate compatible with motion vision. In the present approach, depolarization of the cones was made possible thanks to the arrestin that is still maintained at very late stages of the disease in both RCD models - essential point which was not known in the art before the present invention. This was noticeable in the flicker ERG traces showing responses of the retina during repetitive light stimuli and also by the improved optokinetic reflex of treated mice.

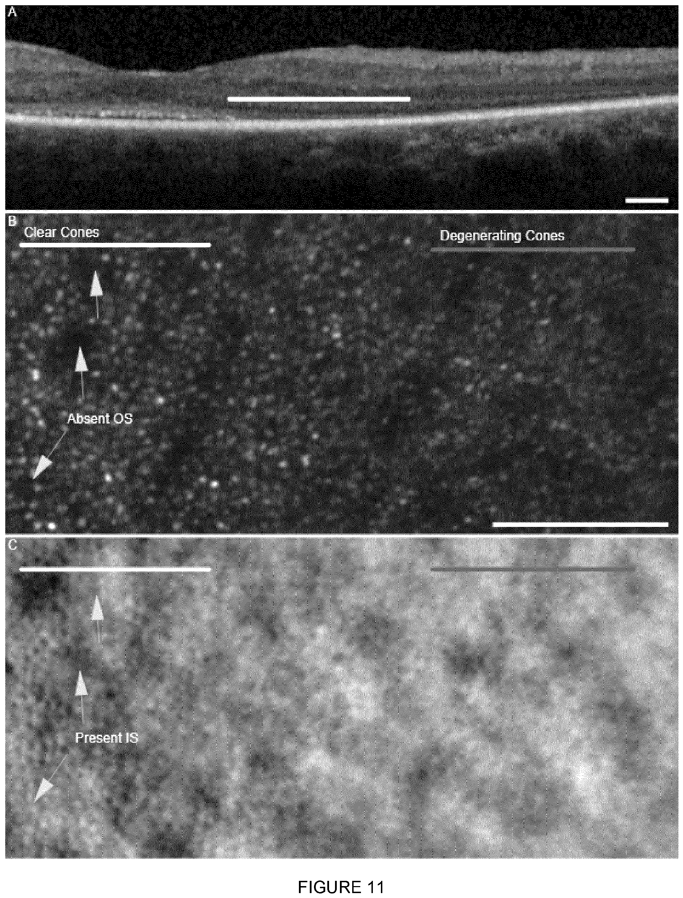

Lastly, the fact that incorporation of GIRK2 enhances existing light responses in cones even prior to complete outer segment loss offers the possibility of implementing this gene therapy in mid stages of the disease. Retinal degeneration in mice is much faster than in humans, thus a few days of therapeutic efficiency in mice is equal to several years in humans. Nonetheless, even when endogenous light responses disappear, retinas expressing GIRK2 are still able to respond to light, generating response amplitudes that correspond to remaining cone numbers. This suggests that patients with remaining foveal cones can benefit from this treatment even if they have no detectable light perception at the beginning of treatment (Figure 11). Thus this approach can be used as long as cone cells remain. Indeed a decrease in the response of treated cones to light stimuli was recorded, which was consistent with decrease in cone numbers and the fact that we did not transduce all cones due to subretinal injection further limited the beneficial effect. AAV vectors showing better lateral spread can be used to increase transduced cone numbers beyond the bleb (Khabou et al., 2018; International application WO 2018134168) [27, 28]. In order to increase the therapeutic window, neurotrophic factors can be implemented alongside the approach of the present invention. Indeed, AAV-mediated secretion of neurotrophic factors such as the rod-derived cone viability factor (RdCVF) have been shown to delay cone cell death and may be combined with GIRK2 mediated sensitization (Byrne et al., 2015) [29].

An object of the present invention is therefore a vector comprising a nucleotide sequence encoding subunit 2 of G-protein-gated inwardly rectifying potassium channel (GIRK2) or a functional derivative thereof. The vector of the present invention can further comprise a nucleotide sequence encoding a mammalian cone opsin. For example, the mammalian cone opsin is a short wavelength cone opsin (SWO), e.g. from *mus musculus* or human cone opsin. Where present in the same vector, the nucleotide sequence encoding GIRK2 or a functional derivative thereof, and the nucleotide sequence encoding a mammalian cone opsin are preferably under the control of a same promoter, in particular a cone-specific promoter such as pR1.7 or a functional variant thereof, or minimal M-opsin promoter, in particular in a pMNTC expression cassette.

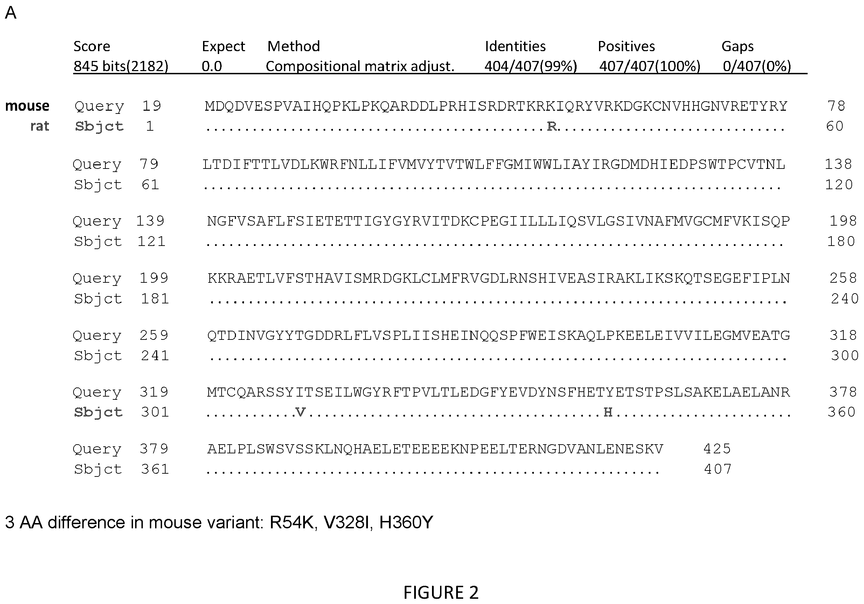

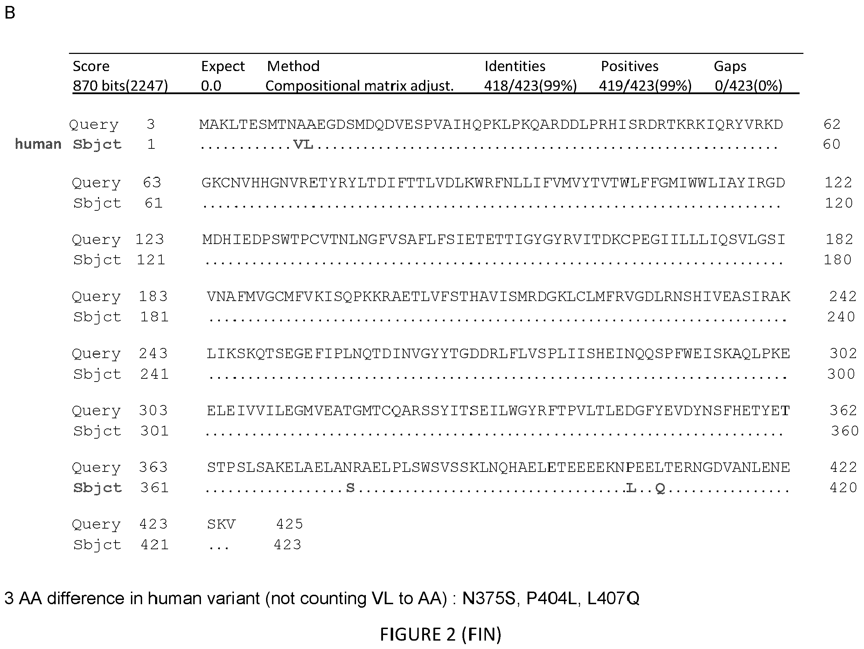

For the purposes of the present invention, "a [GIRK2] functional derivative thereof" means a nucleotide sequence encoding an isoform or variant of GIRK2 which differs by only a few nucleotides compared to the WT form (e.g. mouse) or a nucleotide sequence encoding a truncated GIRK2 (Figure 2), but all of which retain the ability to respond to light when co-expressed with an opsin. For example, a nucleotide sequence encoding GIRK2 or a derivative thereof comprises or consists of the nucleotide sequence SEQ ID NOs: 1, 3 or 5. SEQ ID NO: 4, the polypeptide encoded by SEQ ID NO: 3, comprises a mutation VL to AA at positions 13-14 of the polypeptide sequence which leads to increased cell surface expression of the GIRK2 variant compared to wild-type GIRK2 (Ma et al., 2002) [31].

| Human GIRK2 | |
| --- | --- |
| Nucleotide sequence | ATGGCCAAGCTGACAGAATCCATGACTAACGTCCTGGAGGGCGACTCCATGGA<br>TCAGGACGTCGAAAGCCCAGTGGCCATTCACCAGCCAAAGTTGCCTAAGCAGG<br>CCAGGGATGACCTGCCAAGACACATCAGCCGAGATCGGACCAAAAGGAAAATC<br>CAGAGGTACGTGAGGAAAGACGGAAAGTGCAATGTTCATCACGGCAACGTGAG<br>GGAGACCTATCGCTACCTGACCGATATCTTCACCACATTAGTGGACCTGAAGTG<br>GAGATTCAACCTATTGATTTTTGTCATGGTTTACACAGTGACCTGGCTCTTTTTTG<br>GAATGATCTGGTGGTTGATCGCATACATACGGGGAGACATGGACCACATAGAG<br>GACCCCTCCTGGACTCCTTGTGTTACCAACCTCAACGGGTTCGTCTCTGCTTTTT<br>TATTCTCAATAGAGACAGAAACCACCATTGGTTATGGCTACCGGGTCATCACAG<br>ATAAATGCCCAGAGGGAATTATTCTTCTCTTAATCCAATCTGTGTTGGGGTCCAT<br>TGTCAATGCATTCATGGTGGGATGCATGTTTGTAAAAATCTCTCAACCCAAGAAG<br>AGGGCAGAGACCCTGGTCTTTTCCACCCATGCAGTGATCTCCATGCGGGATGG<br>GAAACTGTGCCTGATGTTCCGGGTAGGGGACCTTAGGAATTCCCACATTGTGGA<br>GGCTTCCATCAGAGCCAAGTTGATCAAATCCAAACAGACCTCGGAGGGGGAGTT<br>CATCCCGTTGAACCAGACGGATATCAACGTAGGGTATTACACGGGGGATGACC<br>GTCTGTTTCTGGTGTCACCGCTGATCATTAGCCATGAAATTAACCAACAGAGTCC<br>TTTCTGGGAGATCTCCAAAGCCCAGCTGCCCAAAGAGGAACTGGAAATTGTGGT<br>CATCCTAGAAGGAATGGTGGAAGCCACAGGGATGACATGCCAAGCTCGAAGCT<br>CCTACATCACCAGTGAGATCCTGTGGGGTTACCGGTTCACACCTGTCCTGACCC<br>TGGAGGACGGGTTCTACGAAGTTGACTACAACAGCTTCCATGAGACCTATGAGA<br>CCAGCACCCCATCCCTTAGTGCCAAAGAGCTGGCCGAGTTAGCCAGCAGGGCA<br>GAGCTGCCCCTGAGTTGGTCTGTATCCAGCAAACTCAACCAACATGCAGAACTG<br>GAGACTGAAGAGGAAGAAAAGAACCTCGAAGAGCAAACAGAAAGAAATGGTGAT<br>GTGGCAAACCTGGAGAATGAATCCAAAGTT (SEQ ID NO : 1)<br>MAKLTESMTNVLEGDSMDQDVESPVAIHQPKLPKQARDDLPRHISRDRTKRKIQRY<br>VRKDGKCNVHHGNVRETYRYLTDIFTTLVDLKWRFNLLIFVMVYTVTWLFFGMIWW<br>LIAYIRGDMDHIEDPSWTPCVTNLNGFVSAFLFSIETETTIGYGYRVITDKCPEGIILLLI<br>QSVLGSIVNAFMVGCMFVKISQPKKRAETLVFSTHAVISMRDGKLCLMFRVGDLRN<br>SHIVEASIRAKLIKSKQTSEGEFIPLNQTDINVGYYTGDDRLFLVSPLIISHEINQQSPF<br>WEISKAQLPKEELEIVVILEGMVEATGMTCQARSSYITSEILWGYRFTPVLTLEDGFY<br>EVDYNSFHETYETSTPSLSAKELAELASRAELPLSWSVSSKLNQHAELETEEEEKNL<br>EEQTERNGDVANLENESKV (SEQ ID NO : 2) |
| Amino acid sequence (423Aa) | MAKLTESMTNVLEGDSMDQDVESPVAIHQPKLPKQARDDLPRHISRDRTKRKIQRY<br>VRKDGKCNVHHGNVRETYRYLTDIFTTLVDLKWRFNLLIFVMVYTVTWLFFGMIWW<br>LIAYIRGDMDHIEDPSWTPCVTNLNGFVSAFLFSIETETTIGYGYRVITDKCPEGIILLLI<br>QSVLGSIVNAFMVGCMFVKISQPKKRAETLVFSTHAVISMRDGKLCLMFRVGDLRN<br>SHIVEASIRAKLIKSKQTSEGEFIPLNQTDINVGYYTGDDRLFLVSPLIISHEINQQSPF<br>WEISKAQLPKEELEIVVILEGMVEATGMTCQARSSYITSEILWGYRFTPVLTLEDGFY<br>EVDYNSFHETYETSTPSLSAKELAELASRAELPLSWSVSSKLNQHAELETEEEEKNL<br>EEQTERNGDVANLENESKV (SEQ ID NO : 2) |

(continued)

| Mouse GIRK2 | | |
| --- | --- | --- |
| Nucleotide sequence | ATGACAATGGCCAAGTTAACTGAATCCATGACTAACGCCGCCGAAGGCGATTCC ATGGACCAGGATGTGGAAAGCCCAGTGGCCATTCACCAGCCAAAGTTGCCTAA GCAGGCCAGGGACGACCTGCCGAGACACATCAGCCGAGACAGGACCAAAAGG AAAATCCAGAGGTACGTGAGGAAGGATGGGAAGTGCAACGTTCACCACGGCAA TGTGCGGGAGACGTACCGATACCTGACGGACATCTTCACCACCCTGGTGGACC TGAAGTGGAGATTCAACCTGTTGATCTTTGTCATGGTCTACACAGTGACGTGGC TTTTCTTTGGGATGATCTGGTGGCTGATTGCGTACATCCGGGGAGATATGGACC ACATAGAGGACCCCTCGTGGACTCCTTGTGTCACCAACCTCAACGGGTTTGTCT CTGCTTTTTTATTCTCCATAGAGACAGAAACCACCATCGGTTATGGCTACCGGGT CATCACGGACAAGTGCCCTGAGGGGATTATTCTCCTCTTAATCCAGTCCGTGTT GGGGTCCATTGTCAACGCCTTCATGGTAGGATGTATGTTTGTGAAAATATCCCAA CCCAAGAAGAGGGCAGAGACCCTGGTCTTTTCCACCCACGCGGTGATCTCCAT GCGGGATGGGAAACTGTGCTTGATGTTCCGGGTGGGGGACTTGAGGAATTCTC ACATTGTGGAGGCATCCATCAGAGCCAAGTTGATCAAGTCCAAACAGACTTCAG AGGGGGAGTTTATTCCCCTCAACCAGACTGATATCAACGTGGGGTACTACACAG GGACGACCGGCTCTTTCTGGTGTCACCATTGATTATTAGCCATGAAATTAACCA ACAGAGTCCCTTCTGGGAGATCTCCAAAGCGCAGCTGCCTAAAGAGGAACTGG AGATTGTGGTCATCCTGGAGGGAATGGTGGAAGCCACAGGAATGACGTGCCAA GCCCGAAGCTCCTACATCACCAGTGAGATCTTGTGGGGTTACCGGTTCACACCT GTCCTAACGCTGGAAGACGGGTTCTACGAAGTTGACTACAACAGCTTCCATGAG ACCTATGAGACCAGCACCCCGTCCCTTAGTGCCAAAGAGCTAGCGGAGCTGGC TAACCGGGCAGAGCTGCCTCTGAGTTGGTCTGTGTCCAGCAAACTGAACCAACA TGCAGAATTGGAGACAGAAGAGGAAGAGAAGAACCCGGAAGAACTGACGGAGA GGAATGGTGACGTGGCAAACCTAGAGAATGAATCCAAAGTT (SEQ ID NO : 3) |
| Amino acid sequence (425Aa) | [two AA difference V$^{13}$L$^{14}$ to] MTMAKLTESMTNAAEGDSMDQDVESPVAIHQPKLPKQARDDLPRHISRDRTKRKIQ RYVRKDGKCNVHHGNVRETYRYLTDIFTTLVDLKWRFNLLIFVMVYTVTWLFFGMIW WLIAYIRGDMDHIEDPSWTPCVTNLNGFVSAFLFSIETETTIGYGYRVITDKCPEGIILL LIQSVLGSIVNAFMVGCMFVKISQPKKRAETLVFSTHAVISMRDGKLCLMFRVGDLR NSHIVEASIRAKLIKSKQTSEGEFIPLNQTDINVGYYTGDDRLFLVSPLIISHEINQQSP FWEISKAQLPKEELEIVVILEGMVEATGMTCQARSSYITSEILWGYRFTPVLTLEDGF YEVDYNSFHETYETSTPSLSAKELAELANRAELPLSWSVSSKLNQHAELETEEEEKN PEELTERNGDVANLENESKV (SEQ ID NO : 4) |

(continued)

| Rat truncated GIRK2 | |
|---|---|
| Nucleotide sequence | ATGGACCAAGACGTGGAAAGCCCAGTGGCCATTCACCAGCCAAAGTTGCCTAA GCAGGCCAGGGATGACCTGCCAAGACACATCAGCCGAGACAGGACCAAAAGGA GAATCCAGAGGTACGTGAGGAAGGATGGGAAGTGTAACGTCCACCACGGCAAC GTGCGGGAGACGTACCGATACCTGACGGACATCTTCACCACCCTGGTGGACCT AAAGTGGAGATTCAACCTATTGATCTTTGTCATGGTCTACACAGTGACGTGGCTT TTCTTTGGGATGATCTGGTGGCTAATTGCATACATCCGGGGAGATATGGACCAC ATAGAGGACCCCTCGTGGACTCCCTGTGTTACCAACCTCAACGGGTTTGTCTCC GCTTTTTTATTCTCAATAGAGACAGAAACCACCATTGGTTATGGCTACAGGGTCA TCACGGACAAGTGCCCAGAAGGAATTATTCTCCTCTTAATCCAGTCCGTGTTGG GGTCCATTGTCAACGCCTTCATGGTAGGATGTATGTTTGTGAAAATATCCCAACC CAAGAAGAGGGCAGAGACCCTGGTCTTTTCCACCCATGCGGTAATCTCCATGCG GGATGGGAAACTATGCCTGATGTTCCGGGTAGGGGACTTGAGGAATTCCCACAT TGTGGAGGCCTCCATCAGAGCCAAGTTGATCAAGTCCAAACAGACTTCAGAGGG GGAGTTCATTCCCCTCAACCAGACGGATATCAACGTAGGGTACTACACCGGGGA TGACCGACTCTTTCTCGTGTCACCGCTGATTATTAGCCATGAAATTAACCAACAG AGTCCCTTCTGGGAGATCTCCAAAGCCCAGCTGCCTAAAGAGGAACTGGAGATT GTGGTCATCCTGGAGGGAATGGTGGAAGCCACAGGAATGACGTGCCAAGCTCG AAGCTCCTACGTCACCAGTGAGATCCTGTGGGGTTACCGGTTCACACCAGTCCT GACACTGGAGGACGGGTTCTATGAAGTTGACTACAACAGCTTCCATGAGACCCA TGAGACCAGCACCCCGTCCCTTAGCGCCAAAGAGCTAGCCGAGCTGGCTAACC GGGCAGAGCTGCCCCTGAGCTGGTCTGTGTCCAGCAAACTGAACCAACATGCA GAACTGGAGACGGAAGAGGAAGAGAAGAACCCGGAAGAACTGACAGAGAGGAA TGGTGATGTGGCAAACCTAGAGAATGAGTCCAAAGTG (SEQ ID NO : 5)<br>**[lacks the first 18AA at N-terminal compared to WT form (NCBI P48550.1)]**<br>MDQDVESPVAIHQPKLPKQARDDLPRHISRDRTKRRIQRYVRKDGKCNVHHGNVR ETYRYLTDIFTTLVDLKWRFNLLIFVMVYTVTWLFFGMIWWLIAYIRGDMDHIEDPSW TPCVTNLNGFVSAFLFSIETETTIGYGYRVITDKCPEGIILLLIQSVLGSIVNAFMVGCM FVKISQPKKRAETLVFSTHAVISMRDGKLCLMFRVGDLRNSHIVEASIRAKLIKSKQT SEGEFIPLNQTDINVGYYTGDDRLFLVSPLIISHEINQQSPFWEISKAQLPKEELEIVVI LEGMVEATGMTCQARSSYVTSEILWGYRFTPVLTLEDGFYEVDYNSFHETHETSTP SLSAKELAELANRAELPLSWSVSSKLNQHAELETEEEEKNPEELTERNGDVANLEN ESKV (SEQ ID NO : 6) |
| Amino acid sequence (407Aa) | [lacks the first 18AA at N-terminal compared to WT form (NCBI P48550.1)]<br>MDQDVESPVAIHQPKLPKQARDDLPRHISRDRTKRRIQRYVRKDGKCNVHHGNVR ETYRYLTDIFTTLVDLKWRFNLLIFVMVYTVTWLFFGMIWWLIAYIRGDMDHIEDPSW TPCVTNLNGFVSAFLFSIETETTIGYGYRVITDKCPEGIILLLIQSVLGSIVNAFMVGCM FVKISQPKKRAETLVFSTHAVISMRDGKLCLMFRVGDLRNSHIVEASIRAKLIKSKQT SEGEFIPLNQTDINVGYYTGDDRLFLVSPLIISHEINQQSPFWEISKAQLPKEELEIVVI LEGMVEATGMTCQARSSYVTSEILWGYRFTPVLTLEDGFYEVDYNSFHETHETSTP SLSAKELAELANRAELPLSWSVSSKLNQHAELETEEEEKNPEELTERNGDVANLEN ESKV (SEQ ID NO : 6) |

Another object of the present invention is a carrier including a vector of the present invention.

According to a particular embodiment of the present invention, the carrier can include a vector comprising a nucleotide sequence encoding subunit 2 of G-protein-gated inwardly rectifying potassium channel (GIRK2) or a functional derivative thereof as described above and a vector comprising a nucleotide sequence encoding a mammalian cone opsin. For example, the mammalian cone opsin is a short wavelength cone opsin (SWO), e.g. from *mus musculus* or human cone opsin. According to an embodiment, the mammalian cone opsin is human Long-wave-sensitive opsin 1 (SEQ ID NO: 16).

| Long-wave-sensitive opsin 1 (OPN1LW) homo sapiens |
|---|
| MVLKAEHTRSPSATLPSNVPSCRSLSSSEDGPSGPSSLADGGLAHNLQDSVRHRILYLSEQ LRVEKASRDGNTVSYLKLVSKADRHQVPHIQQAFEKVNQRASATIAQIEHRLHQCHQQLQEL EEGCRPEGLLLMAESDPANCEPPSEKALLSEPPEPGGEDGPVNLPHASRPFILESRFQSLQ QGTCLETEDVAQQQNLLLQKVKAELEEAKRFHISLQESYHSLKERSLTDLQLLLESLQEEKC RQALMEEQVNGRLQGQLNEIYNLKHNLACSEERMAYLSYERAKEIWEITETFKSRISKLEML QQVTQLEAAEHLQSRPPQMLFKFLSPRLSLATVLLVFVSTLCACPSSLISSRLCTCTMLMLIG LGVLAWQRWRAIPATDWQEWVPSRCRLYSKDSGPPADGP <br> (SEQ ID NO: 16) |

According to a particular embodiment of the present invention, the carrier is for example chosen from solid-lipid nanoparticles, chitosan nanoparticles, liposome, lipoplex or cationic polymer.

According to a particular embodiment of the present invention, the vector of the present invention is a virus, chosen from an adeno-associated virus (AAV), an adenovirus, a lentivirus, an SV40 viral vector. According to a particular embodiment of the present invention, the present invention is equal to or less than 30 nm in size. For example it is an adeno-associated virus (AAV), preferably an AAV8, or an AAV2-7m8 or AAV9-7m8 capsid variant as described in the international application WO 2012145601 [32].

An AAV2-7m8 or AAV9-7m8 capsid variant is an AAV2 or AAV9 virus comprising a 7 to 11 amino acid long insertion peptide in the GH loop of the VP1 capsid protein, wherein the insertion peptide comprises amino acid sequence LGETTRP (SEQ ID NO: 7).

The genomic and polypeptide sequences of various serotypes of AAV, as well as the sequences of the native inverted terminal repeats (ITRs), Rep proteins, and capsid subunits including VP1 protein are known in the art. Such sequences may be found in the literature or in public databases such as GenBank or Protein Data Bank (PDB). See, e.g., GenBank and PDB AF043303 and 1LP3 (AAV-2), AY530579 and 3UX1 (AAV-9 (isolate hu.14)), the disclosures of which are incorporated by reference herein for teaching AAV nucleic acid and amino acid sequences. Exemplary amino acid sequence of wild-type VP1 for AAV9 and AAV2 are shown in SEQ ID NO: 8 and SEQ ID NO:9, respectively.

| wild-type AAV9 VP1 capsid protein |
|---|
| MAADGYLPDWLEDNLSEGIREWWALKPGAPQPKANQQHQDNARGLVLPGYKYLGPGNGL DKGEPVNAADAAALEHDKAYDQQLKAGDNPYLKYNHADAEFQERLKEDTSFGGNLGRAVF QAKKRLLEPLGLVEEAAKTAPGKKRPVEQSPQEPDSSAGIGKSGAQPAKKRLNFGQTGDTE SVPDPQPIGEPPAAPSGVGSLTMASGGGAPVADNNEGADGVGSSSGNWHCDSQWLGDR VITTSTRTWALPTYNNHLYKQISNSTSGGSSNDNAYGYSTPWGYFDFNRFHCHFSPRDWQ RLINNNWGFRPKRLNFKLFNIQVKEVTDNNGVKTIANNLTSTVQVFTDSDYQLPYVLGSAHE GCLPPFPADVFMIPQYGYLTLNDGSQAVGRSSFYCLEYFPSQMLRTGNNFQFSYEFENVPF HSSYAHSQSLDRLMNPLIDQYLYYLSKTINGSGQNQQTLKFSVAGPSNMAVQGRNYIPGPS YRQQRVSTTVTQNNNSEFAWPGASSWALNGRNSLMNPGPAMASHKEGEDRFFPLSGSLIF GKQGTGRDNVDADKVMITNEEEIKTTNPVATESYGQVATNHQSA$Q_{588}Q_{589}$AQTGWVQNQGI LPGMVWQDRDVYLQGPIWAKIPHTDGNFHPSPLMGGFGMKHPPPQILIKNTPVPADPPTAF NKDKLNSFITQYSTGQVSVEIEWELQKENSKRWNPEIQYTSNYYKSNNVEFAVNTEGVYSE PRPIGTRYLTR <br> (SEQ ID NO: 8) |

(continued)

| wild-type AAV2 VP1 capsid protein |
|---|
| MAADGYLPDWLEDTLSEGIRQWWKLKPGPPPPKPAERHKDDSRGLVLPGYKYLGPFNGLD KGEPVNEADAAALEHDKAYDRQLDSGDNPYLKYNHADAEFQERLKEDTSFGGNLGRAVFQ AKKRVLEPLGLVEEPVKTAPGKKRPVEHSPVEPDSSSGTGKAGQQPARKRLNFGQTGDAD SVPDPQPLGQPPAAPSGLGTNTMATGSGAPMADNNEGADGVGNSSGNWHCDSTWMGDR VITTSTRTWALPTYNNHLYKQISSQSGASNDNHYFGYSTPWGYFDFNRFHCHFSPRDWQR LINNNWGFRPKRLNFKLFNIQVKEVTQNDGTTTIANNLTSTVQVFTDSEYQLPYVLGSAHQG CLPPFPADVFMVPQYGYLTLNNGSQAVGRSSFYCLEYFPSQMLRTGNNFTFSYTFEDVPFH SSYAHSQSLDRLMNPLIDQYLYYLSRTNTPSGTTTQSRLQFSQAGASDIRDQSRNWLPGPC YRQQRVSKTSADNNNSEYSWTGATKYHLNGRDSLVNPGPAMASHKDDEEKFFPQSGVLIF GKQGSEKTNVDIEKVMITDEEEIRTTNPVATEQYGSVSTNLQRG$N_{587}R_{588}$QAATADVNTQGV LPGMVWQDRDVYLQGPIWAKIPHTDGHFHPSPLMGGFGLKHPPPQILIKNTPVPANPSTTFS AAKFASFITQYSTGQVSVEIEWELQKENSKRWNPEIQYTSNYNKSVNVDFTVDTNGVYSEP RPIGTRYLTRNL (SEQ ID NO: 9) |

Preferably, the insertion site of the insertion peptide in the GH loop of the VP1 capsid protein is between amino acids 587 and 588 of AAV2 wild-type VP1 capsid protein, between amino acids 588 and 589 of AAV9 wild-type VP1 capsid protein.

According to some embodiments, the insertion peptide has a length of 7 amino acids, 8 amino acids, 9 amino acids, 10 amino acids, or 11 amino acids.

The insertion peptide may comprise one or more spacer amino acids at the N- and/or C-terminus of amino acid sequence LGETTRP (SEQ ID NO: 7). Preferably, the spacer amino acids are selected from the group consisting of Ala, Leu, Gly, Ser, and Thr, more preferably from the group consisting of Ala, Leu, and Gly.

According to an embodiment, the insertion peptide comprises or consists of sequence AALGETTRPA (SEQ ID NO: 10), LALGETTRPA (SEQ ID NO: 11), or GLGETTRPA (SEQ ID NO: 12), preferably comprises or consists of sequence AALGETTRPA (SEQ ID NO: 10) or LALGETTRPA (SEQ ID NO: 11).

According to a particular embodiment, the viral vector, in particular AAV, AAV8, AAV2-7m8 or AAV9-7m8, comprises the polynucleotide of interest (nucleotide sequence encoding GIRK2 or a functional derivative thereof, and/or nucleotide sequence encoding mammalian cone opsin) under the control of a cone-specific promoter, preferably a pR1.7 or a functional variant thereof, or a minimal M-opsin promoter, in particular in a pMNTC expression cassette. In said AAV, the polynucleotide of interest which is operatively linked to the cone-specific promoter, e.g. promoter pR1.7, minimal M-opsin promoter or pMNTC, is preferably flanked by two adeno-associated virus inverted terminal repeats (AAV ITRs).

pR1.7 is a 1.7 kilobases synthetic promoter based on the human red opsin promoter sequence described in Hum Gene Ther. 2016 Jan;27(1):72-82. As used herein, "pR1.7" denotes the promoter of sequence SEQ ID NO:13 and functional variants thereof. "Functional variants" of the pR1.7 promoter typically have one or more nucleotide mutations (such as a nucleotide deletion, addition, and/or substitution) relative to the native pR1.7 promoter (SEQ ID NO: 13), which do not significantly alter the transcription of the polynucleotide of interest. In the context of the present invention, said functional variants retain the capacity to drive a strong expression, in cone photoreceptors, of the polynucleotide of interest. Such capacity can be tested as described by Ye et al. (2016) [33] and Khabou et al. (20183) [34].

Another example of cone-specific promoter which may be used is a minimal M-opsin promoter region such as disclosed in International application WO 2015142941 [35], in particular in SEQ ID NO:55 or SEQ ID NO: 93 as disclosed in WO 2015142941 [35]. Instant sequence SEQ ID NO: 14 is identical to SEQ ID NO: 93 of WO 2015142941 [35].

In an embodiment, the polynucleotide of interest which is placed under the control the minimal M-opsin promoter region, is inserted in a pMNTC expression cassette comprising an optimized enhancer, optimized promoter, optimized 5'UTR, optimized intron, optimized kozak and optimized polyA region (SEQ ID NO:95 of WO 2015142941 [35]).

| pR1.7 promoter |
| --- |
| ggaggctgaggggtggggaaagggcatgggtgtttcatgaggacagagcttccgtttcatgcaatgaaaagagtttggagacgg atggtggtgactggactatacacttacacacggtagcgatggtacactttgtattatgtatattttaccacgatctttttaaagtgtcaaa ggcaaatggccaaatggttccttgtcctatagctgtagcagccatcggctgttagtgacaaagcccctgagtcaagatgacagcag cccccataactcctaatcggctctcccgcgtggagtcatttaggagtagtcgcattagagacaagtccaacatctaatcttccaccct ggccagggccccagctggcagcgagggtggggagactccgggcagagcagagggcgctgacattggggcccggcctggcttg ggtccctctggcctttccccaggggccctctttccttgggggctttcttgggccgccactgctcccgctcctctccccccatcccacccc c tcaccccctcgttcttcatatccttctctagtgctccctccactttcatccacccttctgcaagagtgtgggaccacaaatgagttttcacc tggcctggggacacacgtgcccccacaggtgctgagtgactttctaggacagtaatctgctttaggctaaaatgggacttgatcttct gttagccctaatcatcaattagcagagccggtgaaggtgcagaacctaccgcctttccaggcctcctcccacctctgccacctcca ctctccttcctgggatgtgggggctggcacacgtgtggcccagggcattggtgggattgcactgagctgggtcattagcgtaatcctg gacaagggcagacagggcgagcggagggccagctccggggctcaggcaaggctgggggcttcccccagacaccccactcc tcctctgctggaccccacttcatagggcacttcgtgttctcaaagggcttccaaatagcatggtggccttggatgcccagggaagc ctcagagttgcttatctccctctagacagaagggggaatctcggtcaagagggagaggtcgccctgttcaaggccacccagccag ctcatggcggtaatgggacaaggctggccagccatcccaccctcagaagggacccggtggggcaggtgatctcagaggaggc tcacttctgggtctcacattcttggatccggttccaggcctcggccctaaatagtctccctgggctttcaagagaaccacatgagaaa ggaggattcgggctctgagcagtttcaccacccaccccccagtctgcaaatcctgacccgtgggtccacctgccccaaaggcgg acgcaggacagtagaagggaacagagaacacataaacacagagagggccacagcggctcccacagtcaccgccaccttcc tggcggggatgggtggggcgtctgagtttggttcccagcaaatccctctgagccgcccttgcgggctcgcctcaggagcagggga gcaagaggtgggaggaggaggtctaagtcccaggcccaattaagagatcaggtagtgtagggtttgggagcttttaaggtgaag aggcccgggctgatcccacaggccagtataaagcgccgtgaccctcaggtgatgcgccagggccggctgccgtcggggacag ggctttccatagcc<br>(SEQ ID NO: 13) |

| minimal M-opsin promoter region |
| --- |
| Ccagcaaatccctctgagccgcccccggggggctcgcctcaggagcaaggaagcaaggggtgggaggaggaggtctaagtc ccaggcccaattaagagatcagatggtgtaggatttgggagcttttaaggtgaagaggcccgggctgatcccactggccggtata aagcaccgtgaccctcaggtgacgcaccagggccggctgccgtcggggacagggctttccatagcccag<br>(SEQ ID NO: 14) |

| pMNTC |
| --- |
| cctacagcagccagggtgagattatgaggctgagctgagaatatcaagactgtaccgagtagggggccttggcaagtgtggag agcccggcagctggggcagagggcggagtacggtgtgcgtttacggacctcttcaaacgaggtaggaaggtcagaagtcaaa aagggaacaaatgatgtttaaccacacaaaaatgaaaatccaatggttggatatccattccaaatacacaaaggcaacggata agtgatccgggccaggcacagaaggccatgcacccgtaggattgcactcagagctcccaaatgcataggaatagaagggtgg gtgcaggaggctgaggggtggggaaagggcatgggtgtttcatgaggacagagcttccgtttcatgcaatgaaaagagtttgga gacggatggtggtgactggactatacacttacacacggtagcgatggtacactttgtattatgtatattttaccacgatctttttaaagtg tcaaaggcaaatggccaaatggttccttgtcctatagctgtagcagccatcggctgttagtgacaaagcccctgagtcaagatgac agcagcccccataactcctaatcggctctcccgcgtggagtcatttaggagtagtcgcattagagacaagtccaacatctaatcttc caccctggccagggccccagctggcagcgagggtggggagactccgggcagagcagagggcgctgacattggggcccggcct ggcttgggtccctctggcctttccccaggggccctctttccttgggggctttcttgggccgccactgctcccgctcctctcccccatccca cccccctcacccccctcgttcttcatatccttctctagtgctccctccactttcatccacccttctgcaagagtgtgggaccacaaatgagttt tcacctggcctggggacacacgtgcccccacaggtgctgagtgactttctaggacagtaatctgctttaggctaaaatgggacttga tcttctgttagccctaatcatcaattagcagagccggtgaaggtgcagaacctaccgcctttccaggcctcctcccacctctgccacc tccactctccttcctgggatgtgggggctggcacacgtgtggcccagggcattggtgggattgcactgagctgggtcattagcgtaat cctggacaagggcagacagggcgagcggagggccagctccggggctcaggcaaggctgggggcttcccccagacaccccca ctcctcctctgctggaccccacttcatagggcacttcgtgttctcaaagggcttccaaatagcatggtggccttggatgcccaggga agcctcagagttgcttatctccctctagacagaagggggaatctcggtcaagagggagaggtcgccctgttcaaggccacccagc cagctcatggcggtaatgggacaaggctggccagccatcccaccctcagaagggacccggtggggcaggtgatctcagagga ggctcacttctgggtctcacattcttccagcaaatccctctgagccgccccggggggctcgcctcaggagcaaggaagcaaggg gtgggaggaggaggtctaagtcccaggcccaattaagagatcagatggtgtaggatttgggagcttttaaggtgaagaggcccg ggctgatcccactggccggtataaagcaccgtgaccctcaggtgacgcaccagggccggctgccgtcggggacagggctttcc atagcccaggtaagtatcaaggttacaagacaggtttaaggagaccaatagaaactgggcttgtcgagacagagaagactcttg cgtttctgataggcacctattggtcttactgacatccactttgcctttctctccacaggcccagagaggagacaggccgccacc<br>(SEQ ID NO: 15) |

The promoter and the polynucleotide of interest are operatively linked. As used herein, the term "operatively linked" refers to two or more nucleic acid or amino acid sequence elements that are physically linked in such a way that they are in a functional relationship with each other. For instance, a promoter is operatively linked to a coding sequence if the promoter is able to initiate or otherwise control/regulate the transcription and/or expression of a coding sequence, in which case the coding sequence should be understood as being "under the control of" the promoter. Generally, when two nucleic acid sequences are operatively linked, they will be in the same orientation and usually also in the same reading frame. They will usually also be essentially contiguous, although this may not be required.

According to an embodiment, the vector is an AAV9 (AAV9-7m8-pR1.7) comprising:

- a VP1 capsid protein in which a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
- the polynucleotide of interest (nucleotide sequence encoding GIRK2 or a functional derivative thereof and/or nucleotide sequence encoding mammalian cone opsin) under the control of a pR1.7 promoter.

In said AAV9-7m8, the insertion peptide has a length of 7 amino acids, 8 amino acids, 9 amino acids, 10 amino acids, or 11 amino acids. Preferably, the insertion peptide comprises one or more spacer amino acids at the N- and/or C-terminus of amino acid sequence LGETTRP (SEQ ID NO: 7). Preferably, the spacer amino acids are selected from the group consisting of Ala, Leu, Gly, Ser, and Thr, more preferably from the group consisting of Ala, Leu, and Gly. According to an embodiment, the insertion peptide comprises or consists of sequence AALGETTRPA (SEQ ID NO: 10), LALGETTRPA (SEQ ID NO: 11), or GLGETTRPA (SEQ ID NO: 12); preferably comprises or consists of sequence AALGETTRPA (SEQ ID NO: 10) or LALGETTRPA (SEQ ID NO: 11).

The vectors of the invention are produced using methods known in the art. In short, the methods generally involve (a) the introduction of the AAV vector into a host cell, (b) the introduction of an AAV helper construct into the host cell, wherein the helper construct comprises the viral functions missing from the AAV vector and (c) introducing a helper virus into the host cell. All functions for AAV virion replication and packaging need to be present, to achieve replication and packaging of the AAV vector into AAV virions. The introduction into the host cell can be carried out using standard virology techniques simultaneously or sequentially. Finally, the host cells are cultured to produce AAV virions and are purified using standard techniques such as iodixanol or CsCl gradients or other purification methods. The purified AAV virion is then ready for use.

Another object of the present invention is a pharmaceutical composition comprising the vector or the carrier of the present invention, with a pharmaceutically acceptable carrier, diluent or excipient.

Another object of the present invention is a vector, a carrier or a pharmaceutical composition of the present invention, for use in treating rod-cone dystrophy (RCD).

Rod-cone dystrophy (RCD) is a heterogeneous group of diseases such as Retinitis Pigmentosa (RP), in particular non-syndromic X-linked Retinitis Pigmentosa (XLRP), autosomal recessive RP, autosomal dominant RP. The most common syndromic forms of RCD include Usher syndrome, Bardet-Biedl syndrome, Refsum disease, Bassen-Kornzweig syndrome and Batten disease.

The RCD subject to be treated is a mammal, in particular a non-human or human primate. Particularly, the RCD subject or RCD patient to be treated is a human. The RCD in the mammal may be at an early, intermediate or advanced stage of the disease. In RCD subjects at intermediate or advanced stage of the disease, transduction of the subjects' cones with a nucleotide sequence GIRK2 or a functional derivative thereof is sufficient to achieve vision restoration provided cone opsin and cone arrestin are still expressed in the patients' cone cell bodies. In RCD subjects whose cone cell bodies no longer express cone opsin, transduction of the subjects' cones with a nucleotide sequence GIRK2 or a functional derivative thereof and a mammalian cone opsin is required.

Treatment of RCD may be implemented by administering the vector(s), carrier or pharmaceutical composition of the present invention to the mammal, so as to achieve transduction of cones with the GIRK2 transgene, or GIRK 2 and mammalian cone opsin transgenes.

In other words, another object of the present invention is a method of treating a RCD in a mammal in need thereof, the method comprising administering to the mammal an effective amount of the vector or the carrier of the pharmaceutical composition of the present invention.

Accordingly, in a first embodiment, the vector comprising a nucleotide sequence encoding GIRK2 or a functional derivative thereof, carrier including said vector, or a pharmaceutical composition comprising the vector or carrier is for use in treating rod-cone dystrophy in a RCD mammalian subject whose cone cells still express endogenous cone opsin. According to an embodiment, the vector further comprises a nucleotide sequence encoding a mammalian cone opsin. According to another embodiment, the vector does not comprise a nucleotide sequence encoding a mammalian cone opsin. According to an embodiment, the carrier further includes a vector comprising a nucleotide sequence encoding a mammalian cone opsin. According to another embodiment, the carrier does not include a vector comprising a nucleotide sequence encoding a mammalian cone opsin.

In a second embodiment, the vector comprising a nucleotide sequence encoding GIRK2 or a functional derivative thereof, carrier including said vector, or a pharmaceutical composition comprising the vector or carrier is for use in treating rod-cone dystrophy in an RCD mammalian subject whose cone cells no longer express endogenous cone opsin. According to this embodiment, the vector further comprises a nucleotide sequence encoding a mammalian cone opsin, or the carrier further includes a vector comprising a nucleotide sequence encoding a mammalian cone opsin.

In particular, the vector comprising a nucleotide sequence encoding GIRK2 or a functional derivative thereof, the carrier including said vector, or the pharmaceutical composition comprising the vector or carrier, as described above, are for use in the treatment of rod-cone dystrophy in an RCD subject having a presence of cone photoreceptor cells displaying shortened or absent outer-segments in the outer nuclear layer (ONL).

In particular, the vector comprising a nucleotide sequence encoding GIRK2 or a functional derivative thereof, the carrier including said vector, or the pharmaceutical composition comprising the vector or carrier, as described above, are for use in the treatment of rod-cone dystrophy in an RCD subject having a visual acuity equal or inferior to 6/10, in particular equal or inferior to 5/10, 4/10, 3/10 or 2/10. More particularly the RCD subject has a visual acuity equal or inferior to 2/10.

In particular, the vector comprising a nucleotide sequence encoding GIRK2 or a functional derivative thereof, the carrier including said vector, or the pharmaceutical composition comprising the vector or carrier, as described above, are for use in the treatment of rod-cone dystrophy in an RCD subject having:

- A visual acuity equal or inferior to 6/10, in particular equal or inferior to 5/10, 4/10, 3/10 or 2/10 and
- A presence of cone photoreceptor cells displaying shortened or absent outer segments in the outer nuclear layer (ONL).

In particular, said RCD subject having a presence of cone photoreceptor cells displaying shortened or absent outer-segments in the outer nuclear layer (ONL) and/or having a visual acuity equal or inferior to 6/10, further has an absence or a low light perception. This low to no light perception may be determined by ophthalmologic tests well-known by skilled persons of the art.

Said subject suffering from a rod-cone dystrophy, also called RCD subject, is a mammal, particularly a human.

The visual acuity is expressed in the present application in "tenths" of acuity. The minute of arc (1'), which is the reference for normality, is referred to a fraction of 10.

The visual acuity (VA) is then equal to the inverse of the minimum apparent diameter and is expressed in tenths:

$$VA = \frac{1}{\alpha}$$

where $\alpha$ is the apparent diameter in arc minutes.

$$a = \frac{180 \times 60}{\pi} \cdot \arctan\left(\frac{d'}{D}\right) \simeq 3,44 \cdot \frac{d}{D}$$

wherein

d : minimum distance of discernible points, expressed in mm.
d' : minimum distance of discernible points, expressed in m.
D : observation distance, expressed in meters.

The arctan function is expressed here in radians.

For examples, if $\alpha$ = 1' (one minute of arc), then VA = 10/10 (ten tenths); if $\alpha$ = 2.5' then VA = 4/10.

Different charts exist to measure the visual acuity such as the Monoyer chart and Landolt chart (also called Landolt rings), Parinaud chart or Snellen chart.

A person specialized in eyes and their pathologies, such as an ophthalmologist, perfectly knows how to measure the visual acuity [63-64].

The cone photoreceptor cells displaying shortened or absent outer segments are also called dormant cones.

In other words, patients have the presence of an outer nuclear layer (ONL) filled with cone photoreceptor cells displaying shortened or absent outer-segments or the presence of dormant cones. This implies that the ONL is detectable. The distinguishability of the ONL is determined by examining the structure of the foveal region containing the cone cells.

ONL distinguishability and the presence of cone photoreceptor cells displaying shortened or absent outer segments in the ONL may be determined by in vivo ophthalmic imaging techniques which provide eye scans, such as OCT, AOSLO or OCT combined with AOSLO.

Typically, optical coherence tomography (OCT) scanning may be used. OCT scanning allows to obtain images of the structures of the eye. This is a noninvasive method and does not usually require dilating drops. All that is required from the patient is to keep their eye still while looking at a fixation light inside the machine. OCT is a common method of the state of art [65-68], in particular to observe the ONL and to distinguish if there are cone photoreceptor cells displaying shortened or absent outer-segments [69].

ONL and the presence of dormant cones may also be observed by adaptative optic scanning laser ophthalmoscopy (AOSLO).

For example, AOSLO focuses on the photoreceptor layer on 2°×2° regions of interest identified from the OCT scans. Adaptive optics correct for the optical aberrations of the eye to achieve diffraction limited cell scale resolution in the patient's retina. Typically, the AOSLO is operated in dual channel mode where the confocal channel detects light scattered back on-axis from intact photoreceptors, and the split detection channel detects multiply scattered light emerging from the inner segments. By comparing and combining the appearance of these two channels, intact (inner and outer segment present) and damaged (outer segment absent, or both inner and outer segment absent) cones can be distinguished.

AOSLO is a well-known method of the state of the art to observe the photoreceptor layer of the eye and in particular the cones ([60], [69], [70]).

Treatment of RCD may also be implemented by transducing a mammalian cone precursor cell with vector(s), carrier or pharmaceutical composition of the present invention, and administering the transduced mammalian cone precursor cell to the retina, in particular to the fovea region, of the RCD mammal.

In other words, another object of the present invention is a method of treating a RCD in a mammal in need thereof, the method comprising administering to the mammal an effective amount of mammalian cone precursor cell transduced with the vector or the carrier of the pharmaceutical composition of the present invention.

The invention also relates to a cone precursor cell comprising a heterologous nucleic acid encoding GIRK2 or a functional derivative thereof, or encoding GIRK2 or a functional derivative thereof and a mammalian cone opsin, for use in treating a RCD. Accordingly, it is also provided a method of treating a RCD in a mammal in need thereof, the method comprising administering to the mammal a cone precursor cell comprising a heterologous nucleic acid encoding GIRK2 or a functional derivative thereof, or encoding GIRK2 or a functional derivative thereof and a mammalian cone opsin. As used herein, the term « heterologous nucleic acid » refers to a gene, polynucleotide or nucleic acid sequence that is not in its natural environment.

In particular, said mammal in need is an RCD mammal having:

- A visual acuity equal or inferior to 6/10, in particular equal or inferior to 5/10, 4/10, 3/10 or 2/10; and/or
- A presence of cone photoreceptor cells displaying shortened or absent outer-segments in the outer nuclear layer (ONL).

In particular, said mammal in need further has an absence or a low light perception.

Cone precursor cells are not-fully differentiated, non-dividing cells committed to differentiate into cone cells.

In an embodiment, cone precursor cells are obtained from retina of donor (e.g. cadaver eye donor) or from the RCD subject to be treated, preferably from the RCD subject to be treated. In another embodiment, cone precursor cells are obtained from stem cells, in particular embryonic stem cells, induced pluripotent stem (iPS cells), adult stem cells or fetal stem cells. In another embodiment, cone precursor cells are obtained from differentiated embryonic stem cells. According to one embodiment, embryonic stem cells are non-human embryonic stem cells. According to another embodiment, human embryonic stem cells may be used with the proviso that the method itself or any related acts do not include destruction of human embryos. Preferably cone precursor cells are obtained by differentiation of stem cells, preferably from differentiation of adult stem cells or induced pluripotent stem cells, more preferably from differentiation of induced pluripotent stem cells obtained from somatic cells, e.g. fibroblasts, of the RCD subject to be treated.

Embryonic stem cells are able to maintain an undifferentiated state or can be directed to mature along lineages deriving from all three germ layers, ectoderm, endoderm and mesoderm. Embryonic stem cells can be reprogrammed towards cone photoreceptors by manipulation of key developmental signaling pathways as described in the international application WO 2018055131 [36]. For example, it may be used antagonists of the nodal and wnt pathway in addition to activin-A and serum (Watanabe K et al, 2005) [37], or inhibition of the Notch signaling pathway can be implemented (Osakada F et al., 2009) [38]. Cone precursor cells can be obtained from embryonic stem cells using any protocol known by the skilled person (Osakada F et al., 2008; Amirpour N et al., 2012; Nakano T et al., 2012; Zhu Y et al., 2013; Yanai A et al., 2013; Kuwahara A et al., 2015; Mellough CB et al., 2015; Singh K et al., 2015) [39-46].

Preferably, cone precursor cells are obtained from iPS cells or adult stem cells, more preferably from iPS cells. Induced pluripotent stem (iPS) cells are derived from a non-pluripotent cell, typically an adult somatic cell, by a process known as reprogramming, where the introduction of only a few specific genes are necessary to render the cells pluripotent (e.g. OCT4, SOX2, KLF4 and C-MYC in human cells). One benefit of use of iPS cells is the avoidance of the use of embryonic cells altogether and hence any ethical questions thereof. Photoreceptor precursor cells can be obtained from iPS cells using any differentiation method known by the skilled person.

In particular, photoreceptor precursor cells can be obtained from human iPS cells by a method as disclosed in Garita-Hernandez et al. (2019) [47]. Human iPS are expanded to confluence in iPS medium (e.g. Essential 8™ medium, GIBCO, Life Technologies). After 80% confluence, the medium was switched to a proneural medium (e.g. Essential 6™ medium supplemented with 1 % N2 supplement (100X); GIBCO, Life Technologies). The medium was changed every 2-3 days. After 4 weeks of differentiation, neural retina-like structures grew out of the cultures and were mechanically isolated. Pigmented parts, giving rise to RPE were carefully removed. The extended 3D culture in Maturation medium (DMEM/F-12 medium supplemented with 2% B-27™ Supplement (50X), serum free, and 1% MEM Non-Essential Amino Acids Solution (100X) ; GIBCO, Life Technologies) allowed the formation of retinal organoids. Addition of 10 ng/ml Fibroblast growth factor 2 (FGF2, Preprotech) at this point favoured the growth of retinal organoids and the commitment towards retinal neurons instead of RPE lineage. In order to promote the commitment of retinal progenitors towards photoreceptors, Notch signalling was specifically blocked for a week starting at day 42 of differentiation using the gamma secretase inhibitor DAPT (10 $\mu$M, Selleckchem). Floating organoids were cultured in 6 well-plates (10 organoids per well) and medium was changed every 2 days.

Photoreceptor precursor cells can also be obtained from human iPS cells using any other protocol known by the skilled person (Lamba, Osakada and colleagues: Lamba et al., 2006; Lamba et al., 2010; Osakada et al., 2009; Meyer JS et al., 2009; Meyer JS et al., 2011; Mellough CB et al., 2012; Boucherie C et al., 2013; Sridhar A et al., 2013; Tucker BA et al., 2013; Tucker BA et al., 2013; Eichman S et al., 2014; Zhong X et al., 2014; Wang X et al., 2015) [48, 49, 38, 50-59].

The cone precursor cells comprise a heterologous nucleic acid encoding i) GIRK2 or a functional derivative thereof, or ii) encoding GIRK2 or a functional derivative thereof and a mammalian cone opsin. Where the cone precursor cells comprise a heterologous nucleic acid encoding GIRK2, or a functional derivative thereof, and a mammalian cone opsin, the cone precursor cells either comprise i) a heterologous nucleic acid encoding both GIRK2, or a functional derivative thereof, and a mammalian cone opsin, or ii) a heterologous nucleic acid encoding GIRK2, or a functional derivative thereof, and another heterologous nucleic acid encoding a mammalian cone opsin.

Said cone precursor cells may be prepared by introducing into said cone precursor cells said heterologous nucleic acid(s), or an expression cassette or vector comprising said nucleic acid(s), by any method known to the skilled person. According to an embodiment, a cone precursor cell comprising a heterologous nucleic acid encoding GIRK2 or a functional derivative thereof, or encoding GIRK2 or a functional derivative thereof and a mammalian cone opsin, is prepared by infecting the cone precursor cell with a viral vector as described above, in particular with an AAV vector, preferably the AAV8, AAV2-7m8 or AAV9-7m8.

In another aspect, the invention therefore further refers to a method of preparing a cone precursor cell comprising a heterologous nucleic acid encoding GIRK2 or a functional derivative thereof, or encoding GIRK2 or a functional derivative thereof and a mammalian cone opsin, said method comprising infecting cone precursor cells with a viral vector or carrier according to the invention, and recovering infected cone precursor cells.

The vector, carrier, or pharmaceutical composition, or cone precursor cells may be administered by any suitable route known to the skilled person, in particular by intravitreal or subretinal administration, for the treatment of RCD as above mentioned.

The fovea is a small region in the central retina of primates of approximately equal to or less than 0.5 mm in diameter that contains only cone photoreceptor cells, and highest density of cones in the whole retina. The fovea dominates the visual perception of primates by providing high-acuity color vision. The highest density of cones is found at the center of the fovea (<0.3 mm from the foveal center), devoid of rod photoreceptors. Cone density decreases by up to 100-fold with distance from the fovea.

Cone cells in the fovea are the primary targets of gene therapies aiming to treat inherited retinal diseases like retinitis pigmentosa. Usually, viral vectors encoding therapeutic proteins are injected "subretinally", i.e. into the subretinal space between the photoreceptors and the retinal pigment epithelium (RPE) cells in order to provide gene delivery to cones.

The subretinal delivery leads to the formation of a "bleb", which refers to a fluid-filled pocket within the subretinal space of the injected eye. In this approach, gene delivery is limited to cells that contact the local bleb of injected fluid. Retinal detachment, and in particular foveal detachment, that occurs during subretinal injections is a concern in eyes with retinal degeneration.

Advantageously, when the vector is an AAV9-7m8 vector (in particular AAV9-7m8-pR1.7 vector), the vector (or carrier of pharmaceutical composition comprising said vector) can be administered by a distal subretinal injection, or in the periphery of the fovea, and then spread laterally to reach the foveal region. According to an embodiment the bleb formed is greater than or equal to 0.5 millimeters away from the center of the fovea, without detaching the foveal region.

In particular, subretinal injection of AAV9-7m8 vector (in particular AAV9-7m8-pR1.7 vector) can be performed a) in a region adjacent to the superior or inferior temporal branch of retinal artery; b) at a distance of 2-3 optic disk diameter away from the center of the fovea; and c) at a position localized in the geometric shape, preferably quadrilateral, delineated by the branches of temporal retinal artery and temporal retinal vein, usually between the 3rd and 4th anterior venous crossings (see Figure 13). Preferably, injection is performed at a position forming an angle comprised between -10° and + 10° with the vertical axis of the retina passing through the center of the fovea. In an embodiment, said AAV9-7m8 viral vector is formulated in a solution and 50 to 100 $\mu$L of solution are injected continuously in 20 to 30 seconds. In an embodiment, said AAV9-7m8 viral vector is formulated in a solution at a concentration of $1\times10^{10}$ to $1\times10^{12}$ vg/mL (viral genome/mL), preferably of $0.5\times10^{11}$ to $5\times10^{11}$ vg/mL, still preferably of $1\times10^{11}$ vg/mL.

Preferably, the cone precursor cells are administered by intraocular injection, preferably by subretinal space injection, more preferably by injection between the neural retina and the overlying PE. The amount of cone precursor cells to be administered may be determined by standard procedure well known by those of ordinary skill in the art. Physiological data of the patient (*e.g.* age, size, and weight) and type and severity of the disease being treated have to be taken into account to determine the appropriate dosage. The cone precursor cells may be administered as a single dose or in multiple doses. In particular, each unit dosage may contain, from 100,000 to 300,000 cone precursor cells per $\mu$l, preferably from 200,000 to 300,000 cone precursor cells per $\mu$l.

Another object of the present invention is a nucleotide sequence encoding subunit 2 of G-protein-gated inwardly rectifying potassium channel (GIRK2) or a derivative thereof as described above, for use as a medicament. In particular said nucleotide sequence is useful for treating rod-cone dystrophy (RCD). In other words, another object of the present invention is a method of treating a RCD in a mammal in need thereof, the method comprising administering to the mammal an effective amount of a nucleotide sequence encoding subunit 2 of G-protein-gated inwardly rectifying potassium channel (GIRK2) or a derivative thereof as described above. According to an embodiment, the polynucleotide sequence encoding subunit 2 of G-protein-gated inwardly rectifying potassium channel (GIRK2) or a derivative thereof is under the control of the pR1.7 promoter or of a functional variant of said promoter.

**Brief description of the figures**

Figure 1 represents phototransduction cascade (A) normal phototransduction cascade (B) short phototransduction cascade with an animal opsin and GIRK2 channel. PDE: phosphodiesterase. CNG: cyclic-nucleotic gated channels. cGMP: cyclic guanosine monophosphate.

Figure 2 represents alignments of GIRK2 (A) rat truncated GIRK2 vs mouse GIRK2 (B) mouse GIRK2 vs human GIRK2.

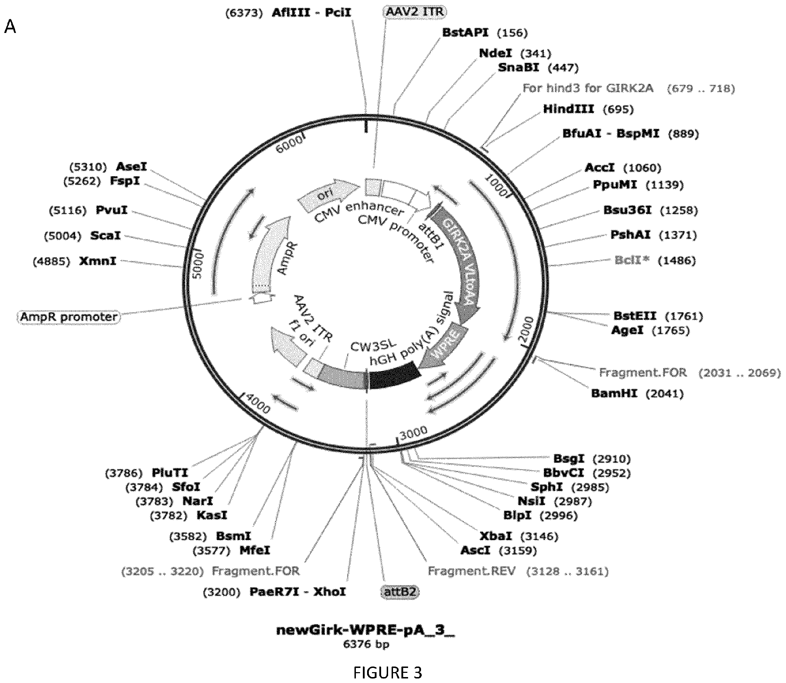

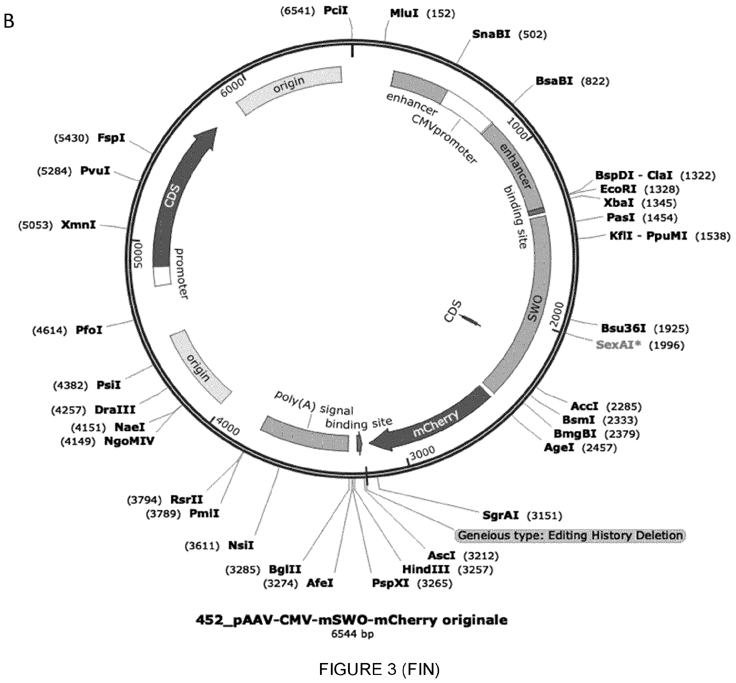

Figure 3 represents plasmids (A) CMV-GIRK2-GFP and (B) CMV-SWO-m Cherry.

Figure 4 represents what remained in the phototransduction cascade in rd10 mice using immunohistochemistry (A-D) retinal cross-section of a control WT mouse stained with (A) opsin, (B) transducin, (C) PDE and (D) cone arrestin. (E-H) retinal cross-section of a rd10 mouse at P14 stained with (E) opsin, (F) transducin, (G) PDE and (H) cone arrestin. (I-L) Retinal cross-section of a rd10 mouse at P150 stained with (I) opsin, (J) transducin, (K) PDE and (L) cone arrestin. ONL: outer nuclear layer. INL: inner nuclear layer. GC: ganglion cells. Scale bar is 50$\mu$m. Inset scale bar is 25$\mu$m.

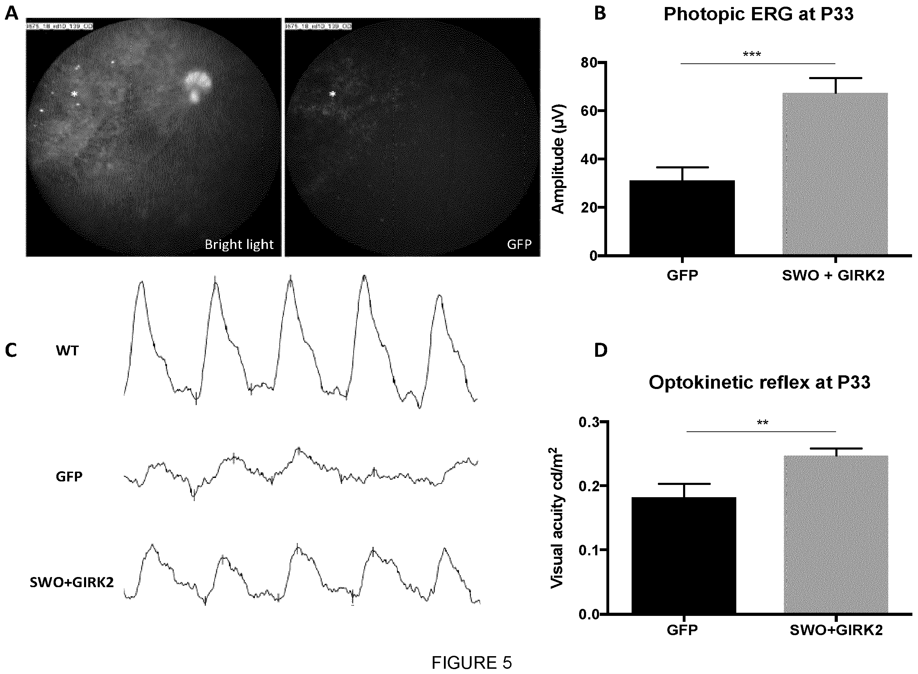

Figure 5 represents preliminary data. (A) Eye fundus of GIRK2-GFP expression in rd10 mouse one week post-injection *(*site of injection)* (B) Photopic ERG amplitude in rd10 mice at P33, injected with AAV-SWO-tdTomato and AAV-GIRK2-GFP. Control mice are injected with AAV-GFP (n=12). *P=0,0002.* (C) Representative flickers ERG at P33. (D) Measure of the visual acuity by optokinetic test in rd10 mice, injected with AAV-SWO-tdTomato and AAV-GIRK2-GFP. Control mice are injected with AAV-GFP. Control mice were injected with AAV-GFP (n=8).

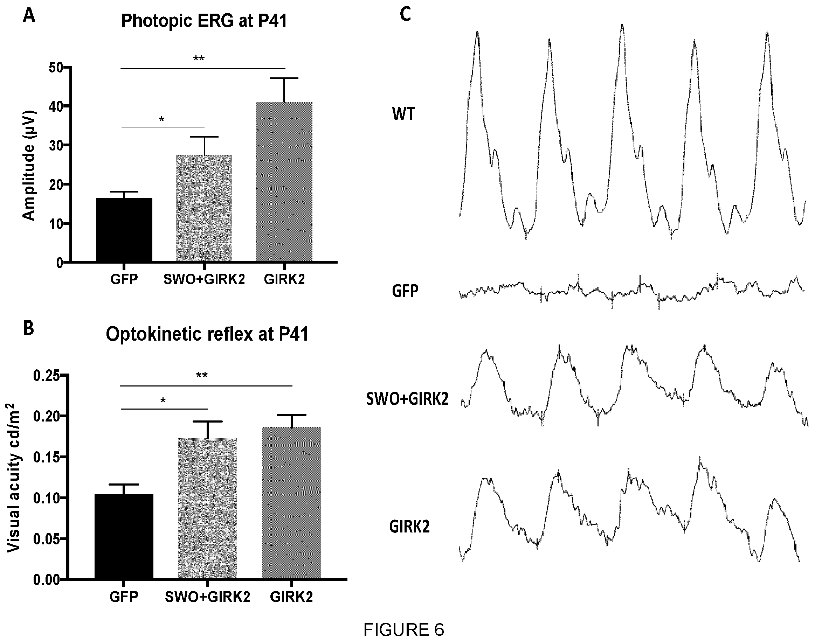

Figure 6 represents GIRK2-mediated vision. (A) Photopic ERG amplitude in rd10 mice at P41, injected with AAV-SWO-tdTomato and/or AAV-GIRK2-GFP. Control mice are injected with AAV-GFP (n=12). $P_{SWO+GIRK2}$ = *0,0381 and* $P_{GIRK2}$=*0,0021.* (B) Measure of the visual acuity by optokinetic test in rd10 mice, injected with AAV-SWO-tdTomato and/or AAV-GIRK2-GFP. Control mice are injected with AAV-GFP. Control mice were injected with AAV-GFP (n=7). (C) Representative flickers ERG at P41.

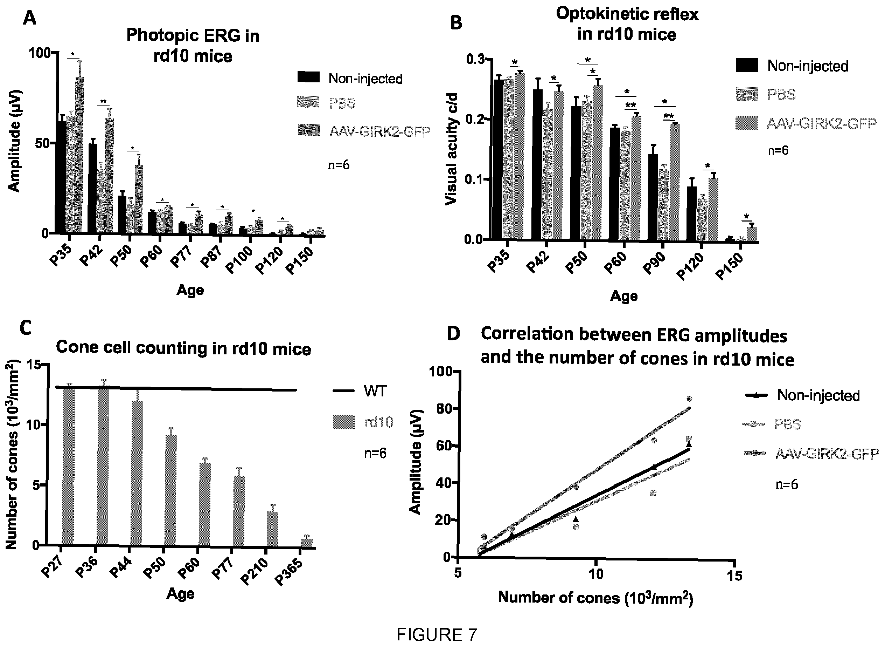

Figure 7 represents long term efficiency. (A) Photopic ERG amplitude in rd10 mice, injected with AAV-GIRK2-GFP. Control mice are injected with PBS (n=6). (B) Measure of the visual acuity by optokinetic test in rd10 mice, injected with AAV-GIRK2-GFP. Control mice are injected with PBS (n=6). (C) Number of cones of wild-type mice and non-injected rd10 mice over time (n=6). *Pvalue* $_{(P50-P365)}$ = 0.0022. (D) Linear regression correlation between the ERG amplitudes and the number of cones in rd10 mice (n=6). *Pvalue* $_{non-injected}$ = 0,0482. *Pvalue* $_{AAV-GIRK2-GFP}$ = 0,0007. *Pvalue* $_{PBS}$ = 0,0104.

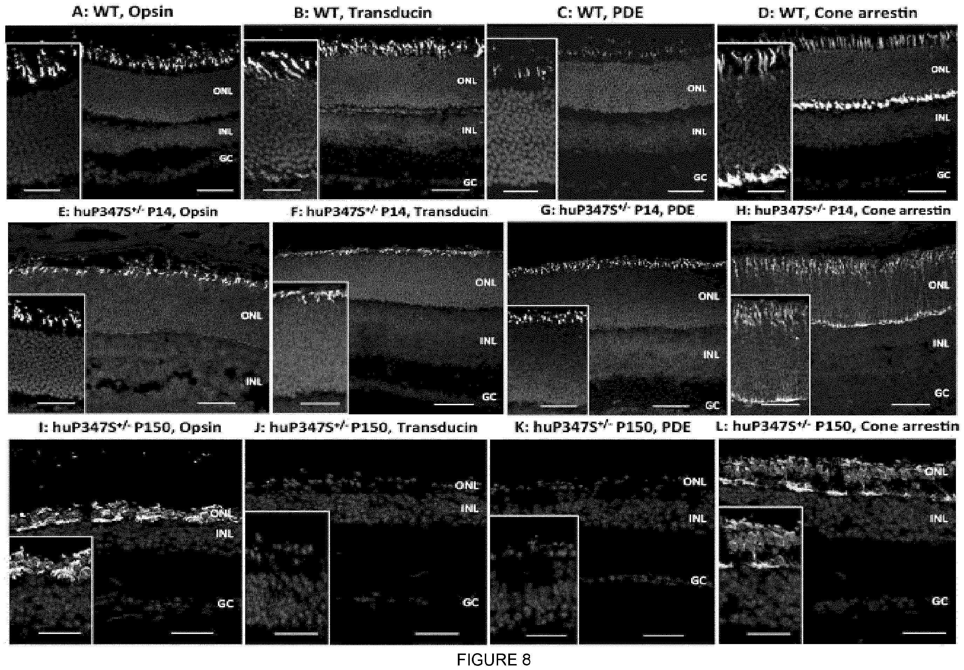

Figure 8 represents what remained in the phototransduction cascade in huP347S$^{+/-}$ mice using immunohistochemistry. (A-D) Retinal cross-section of a control WT mouse stained with (A) opsin, (B) transducin, (C) PDE and (D) cone arrestin. (E-H) retinal cross-section of a huP347S$^{+/-}$ mouse at P14 stained with (E) opsin, (F) transducin, (G) PDE and (H) cone arrestin. (I-L) retinal cross-section of a huP347S$^{+/-}$ mouse at P150 stained with (I) opsin, (J) transducin, (K) PDE and (L) cone arrestin. ONL: outer nuclear layer. INL: inner nuclear layer. GC: ganglion cells. Scale bar is $50\mu m$. Inset scale bar is $25\mu m$.

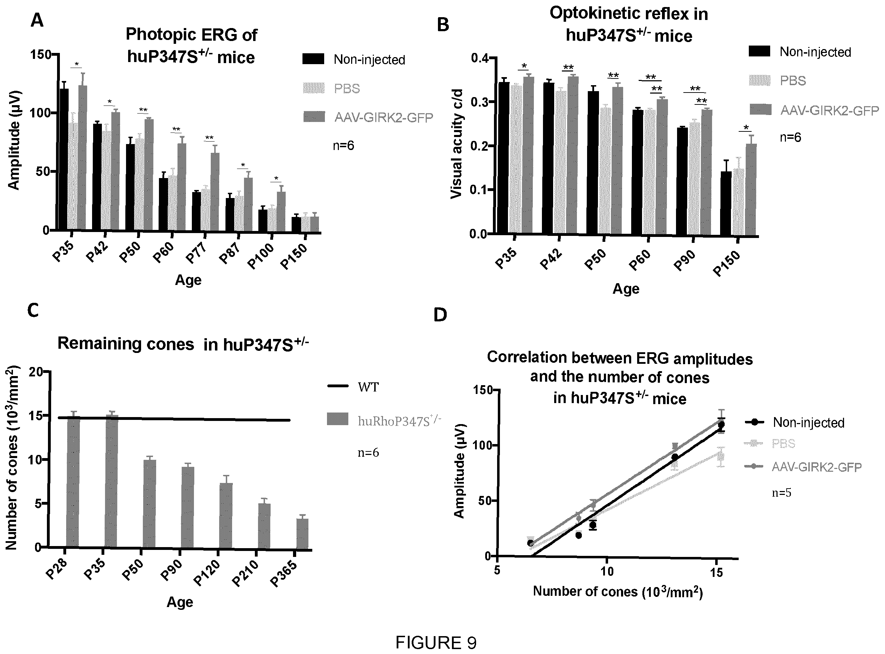

Figure 9 represents universality of the approach. (A) Photopic ERG amplitude in huP347S$^{+/-}$ mice, injected with AAV-GIRK2-GFP. Control mice are injected with PBS (n=6). (B) Measure of the visual acuity by optokinetic test in huP347S$^{+/-}$ mice, injected with AAV-GIRK2-GFP. Control mice are injected with PBS (n=6). (C) Number of cones of wild-type mice and non-injected huP347S$^{+/-}$ mice over time (n=6). *Pvalue* $_{(P50-P365)}$ = 0,0022. (D) Linear regression correlation between the ERG amplitudes and the number of cones in huP347S$^{+/-}$ mice (n=5). *Pvalue* $_{non-injected}$ = 0,0313. *Pvalue* $_{AAV-GIRK2-GFP}$ = 0,0146. *Pvalue* $_{PBS}$ = 0,0497.

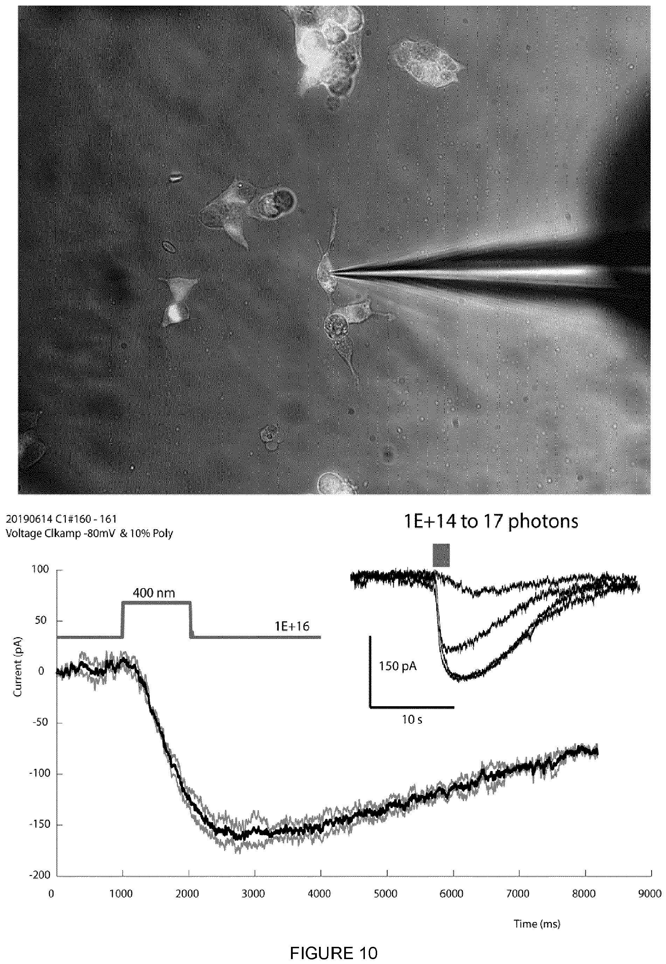

Figure 10 represents the efficiency of the mouse GIRK2 in HEK cells transfected with two plasmids: CMV-SWO-mCherry and CMV-GIRK2-GFP.

Figure 11 represents phenotyping of a normal volunteer and retinitis pigmentosa patients for eligible patient population. Upper panel (A) shows the fundus and OCT images of the back of the eye in a normal individual along with adaptive optics images of cone dominated regions of the retina. Middle panel (B) shows a pie-chart distribution of advanced RCD patients. Lower panel (C) represent OCT and AOSLO images of different patients. AOSLO confocal (up) and AOSLO split detection (low) in vivo retinal images of a patient with retinitis pigmentosa (age 77, male). Acquired fields are located at the transition between regions of presumed dormant cones (i.e. morphologically intact - as indicated by the IS/OS line visible in OCT, clear inner segment mosaic visible in AOSLO split detection, and clear cone mosaic indicating intact inner and outer segments in AOSLO confocal - though with reduced function according to the patient's visual acuity; yellow bars) and damaged or absent cones (indicated by the absent IS/OS line in OCT, and the blurred inner segment and cone mosaics in AOSLO split detection and confocal respectively; red bars). Arrows indicate cones that appear to be degenerating, with absent OS in confocal but present IS in split detection. Scale bars, $200\mu m$.

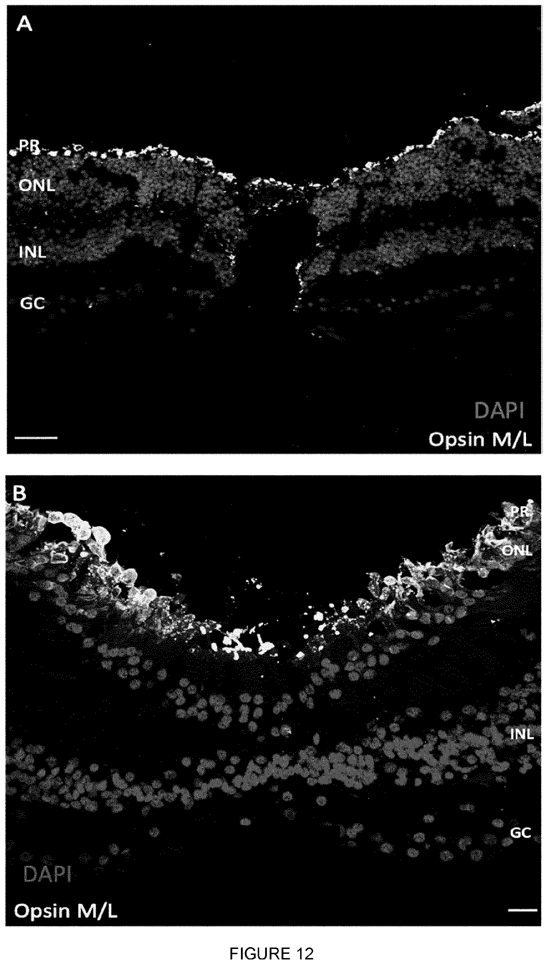

Figure 12 represents immunohistochemistry labeling cone phototransduction cascade proteins in normal and RP human retina. (A) Retinal cross-section of a 86 years old control human retina (20x). (B) Retinal cross-section of a 75 years old human retina affected by retinitis pigmentosa (RP) and having night blindness and loss of peripheral vision (40x). (A-B) stained with Opn1mw, (bright grey) and nuclear stain DAPI (dark grey). ONL: outer nuclear layer. INL: inner nuclear layer. GC: ganglion cells. Scale bar is $50\mu m$. Inset scale bar is $25\mu m$.

Figure 13: Localization of subretinal injection sites to deliver the AAV solution under the retina, close to the fovea but without foveal detachment.

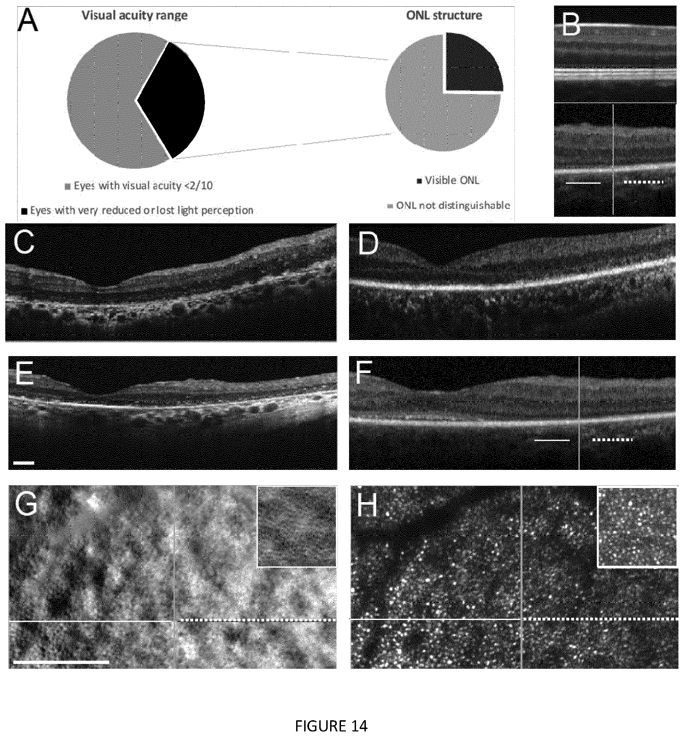

Figure 14 represents the phenotyping of retinitis pigmentosa patients to define an patient population for GIRK2 gene therapy. (A) Proportion of retinitis pigmentosa patients with very reduced or lost light perception and have a detectable ONL filled with diminished outer-segment cone photoreceptor cells. (B) Zooms on OCT scans of a healthy retina (top) versus the patient shown in (F-H) suffering from retinitis pigmentosa (bottom). The vertical light grey line on the OCT cross section marks the transition between a zone with external limiting membrane, implying some residual outer segment structure (horizontal white bar) and a zone with absent external limiting membrane (horizontal dotted white bar). (C-F) Representative OCT scans from the left eye of four RP patients (aged from 32 to 77 years old) suffering from retinitis pigmentosa. (G-H) AOSLO images over the same zone shown in (B) on a retinitis pigmentosa patient, with inserts from a healthy subject for comparison. Split detection (G) and confocal (H) modalities show transition from clear cone mosaics (horizontal white bar) suggesting cones with some residual structure maintaining them in a mosaic packing, to presumed damaged cones with no clear mosaic visible (horizontal dotted white bar) over this region. Scale bar, $200\mu m$.

## EXAMPLES

## MATERIAL AND METHODS

## 1. Animals

C57BL/6j$^{rd10/rd10}$(rd10) mice were used in these experiments. They have a mutation on the rod PDE gene leading to a dysfunctional phototransduction cascade and a rod-cone dystrophy. The second model used is the huRhoP347S[+/-] mouse. The homozygous strand of this mouse present a KO of mouse rhodopsin (mRho) gene and a KI of human rhodopsin (huRho) with a mutation (P347S) (Millington-Ward et al., 2011) [30]. The homozygous males were crossed C57BL/6j (wild-type) females to obtain heterozygous mice. These mice have a similar phenotype as the rd10 mice but the degeneration rate is lower.

## 2. AAV injections

Mice were first anesthetised with intraperitoneal injections of 0.2 ml/20g ketamine (Ketamine 500, Vibrac France) and xylazine (Xylazine 2%, Rompun) diluted in 0.9% NaCl. Eyes were dilated with 8% Neosynephrine (Neosynephrine Faure 10%, Europhta) and 42% Mydriaticum (Mydriaticum 0.5%, Thea) diluted in 0.9% NaCl.

A total volume of $1\mu$l of vector solution was injected subretinally. Fradexam, an ophthalmic ointment, was applied after injection. The list of injected viral vectors is presented below:

| Injection Table | | | | |
|---|---|---|---|---|
| Mice | Eyes | viral vector injected | viral vector titration | volume injected |
| rd 10 | both | AAV8-mCAR-GFP | $10^{11}$ rAAV particles | 1 $\mu$l for all conditions |
| rd 10 | both | AAV8-mCAR-G I RK2-G FP+ AAV8-mCAR-SWO-tdTomato | | |
| rd 10 | both | AAV8-mCAR-GIRK2-GFP | | |
| rd 10 | right | PBS | | |
| rd10 | left | AAV8-PR1.7-GIRK2-GFP | $5.10^8$ rAAV particles | |
| huRhoP347S[+/-] | right | PBS | | |
| huRhoP347S[+/-] | left | AAV8-PR1.7-GIRK2-GFP | $5.10^8$ rAAV particles | |

## 3. Eye fundus examination

One week after subretinal injection, mice were anesthetised by isofluorane inhalation. Eyes were dilated and then protected with Lubrithal eye gel (VetXX). Fundus imaging was performed with a fundus camera (Micron III; Phoenix research Lab) equipped with specific filters to monitor GFP or tdTomato expression in live anesthetised mice.

## 4. Electroretinography (ERG) recordings

To evaluate retinal function, electroretinography recordings (ERG) were recorded (espion E2 ERG system; Diagnosys). Several tests were performed at different time points after injections of the viral vectors. Mice were anesthetised with intraperitoneal injections of 0.2 ml/20g ketamine (Ketamine 500, Vibrac France) and xylazine (Xylasine 2%, Rompun) diluted in 0.9% NaCl. Mice were then placed on a heated pad at 37°C. Eyes were dilated with Neosyhephrine (Neosynephrine Faure 10%, Europhta) and Mydriaticum (Mydriaticum 0.5%, Thea) diluted in 0.9% NaCl. Eyes were protected with Lubrithal eye gel before putting electrodes on the corneal surface of each eye. The reference electrode was inserted under the skin into the forehead and a ground electrode under the skin in the back.

ERG recordings were done under two conditions: (i) photopic condition, which reflects con-driven light responses - 6ms light flashes were applied every second during 60 seconds at increasing light intensities (0.1/1/10/50cd s/m) after an adaptation of 5 minutes at 20cd s/m - and (ii) flicker condition, which are rapid frequency light stimuli that reflect cone function (70 flashes at 10Hz et 1cd s/m).

Graph and statistical analysis were performed using GraphPad.

## 5. Optokinetic test

Visual acuity was measured using an optokinetic test scoring the head turning movement of a mouse placed in front of moving bars. Testing was performed using a computer-based machine consisting of four computer monitors arranged in a square to form an optokinetic chamber. A computer program was designated to generate the optokinetic stimuli, consisting of moving alternate black and white stripes. The spatial frequency is ranging from 0.03 to 0.6 cyc/deg. The program enabled modulation of stripe width and direction of bar movement.

## 6. Immunohistochemistry and confocal imaging

Animals were sacrificed by $CO_2$ inhalation, and the eyes were enucleated and fixed in 4% paraformaldehyde-PBS for 1h at room temperature. The eyes were dissected either as eyecups for immunohistochemistry or prepared as flat mounts for cell counting. The eyecups were then cryoprotected with a gradient of PBS-Sucrose 10% for 1h and then in PBS-Sucrose 30% overnight. The eyecups were embedded in OCT and 12 $\mu$m thick cryostat sections (ThermoFisher) were cut and mounted on glass slides. The sections were washed in PBS (3x5 mins) and stained against different antibodies (see table below) and DAPI (1 :2000). The sections were finally washed in PBS, mounted in Fluoromount Vactashield (Vector Laboratories) and coverslipped for imaging using laser-confocal microscopy (Olympus IX81). For flat-mount retina stainings, the protocol is the same except that the tissue was not cryoprotected. Images were analysed using FIJI software.

| Antibody table | | |
|---|---|---|
| Target | Host | Clonality/Conjugated |
| PRIMARY ANTIBODIES | | |
| Red/Green opsin (M/L opsin) | Rabbit | Polyclonal |
| Mouse Cone arrestin | Rabbit | Polyclonal |
| PDE6C | Rabbit | Polyclonal |
| GNAT2 | Rabbit | Polyclonal |
| PNA-Lectin | Conjugated with FITC | |
| SECONDARY ANTIBODIES | | |
| Anti-rabbit | Donkey | AF 546 |

## 7. Cell counts

Flat mount retinas of rd10 and huRhoP347S[+/-] mice were stained using antibodies against mouse cone arrestin - mCAR (1:10000) and DAPI (1:2000). The double stained cells counted at different ages. Retinas from 5 animals (n=10) were used for each age and were oriented dorso-ventrally and naso-temporally. Serial optical sections were obtained to cover the thickness of the entire outer nuclear layer (ONL). Two scanning areas of 211.97 $\times$ 211.97 $\mu$m were made in each of the four regions in all retinas. Counts of cone cells were performed manually using the FIJI software by the reconstruction of the images (z stack) covering the entire thickness of the ONL. Average density values of each retina were calculated to obtain the number of cone cells per mm$^2$ at different ages.

## 8. *In vitro* test of the efficiency of mouse GIRK2

HEK cells were transfected with two plasmids: CMV-SWO-mCherry and CMV-GIRK2-GFP (Figure 3) according to a well-known procedure in the art. HEK293 cells were cultured and recorded in dark room conditions after transfection. Cells were placed in the recording chamber of a microscope equipped with a 25x water immersion objective (XLPlanN-25 $\times$ -W-MP/NA1.05, Olympus) at 36 °C in oxygenated (95% O2/5% CO2) Ames medium (Sigma-Aldrich) enriched with an addition of 1 mM9-cis-retinal. KGluconate was added to the external solution in order to get a high extracellular potassium concentration leading to a cell potassium reversal potential of -40mV.

For Whole-cell recordings, the Axon Multiclamp 700B amplifier (Molecular Device Cellular Neurosciences) was used, GIRK-mediated K+-currents were recorded in voltage-clamp configuration at -80 mV, using borosilicate glass pipettes (BF100-50-10, Sutter Instrument) pulled to 5M$\Omega$ and filled with 115 mMK Gluconate, 10 mM KCl, 1 mM MgCl2, 0.5 mM CaCl2, 1.5 mM EGTA, 10 mM HEPES, and 4 mM ATP-Na2 (pH 7.2).

During experiments, a CCD camera (Hamamatsu Corp.) was used to visualize cells using a trans-illuminated infrared-light. A monochromatic light source (Polychrome V, TILL photonics) was used to stimulate cells during electrophysiological experiments with light flashes at 400 nm.

### 9. Patient eye fundus imaging

Volumes of optical coherence tomography (OCT) (Spectralis, Heidelberg Engineering, Germany) b-scans were acquired in patients covering the macular region[K1]. Adaptive optics scanning laser ophthalmoscopy (AOSLO) (Roorda et al., 2002) [60] was used to image cone photoreceptor mosaic at cell resolution. The AOSLO device used, focused on the photoreceptor layer on 2°×2° regions of interest identified from the OCT scans. Adaptive optics corrected for the optical aberrations of the eye to achieve diffraction limited cell scale resolution in the patient's retina. The AOSLO was operated in dual channel mode where the confocal channel detects light scattered back on-axis from intact photoreceptors, and the split detection channel detects multiply scattered light emerging from the inner segments. By comparing and combining the appearance of these two channels, intact (inner and outer segment present) and damaged (outer segment absent, or both inner and outer segment absent) cones could be distinguished.

In other words, AOSLO allows simultaneous imaging over a 2-degree field of view of intact cones with both inner and outer segments (IS, OS) from light scattered along the optical axis (confocal mode) and inner segments (IS) from multiply scattered light scattered off axis (split detection mode). This allows us to evaluate cone presence and health, with differential imaging of IS versus IS+OS for each cone.

## RESULTS

### 1. The changes in the phototransduction cascade in degenerating cones

The phototransduction cascade was first analysed in the rd10 mouse model by studying its components using immunohistochemistry, at different time points during retinal degeneration. Immunofluorescence staining was performed against cone opsin, transducing, phosphodiesterase and cone arrestin proteins of the phototransduction cascade that interact directly with cone opsin.

Figure 4 shows that only the cone opsin and arrestin were still expressed and localized around the cone cell body at late stage of the disease.

### 2. Cone opsin and GIRK2-mediated vision restoration

Based on immunohistochemistry and previous findings with cone opsins expressed in neurons, it was first studied why delivering a mouse short wavelength cone opsin (SWO) fused with tdTomato and GIRK2 fused with GFP using two AAV vectors mixed in equimolar ratios would enhance cone cell's response to light. Thus two AAVs were injected subretinally to degenerating rd10 mouse retinas at p15 (Figure 5A). This led to a significant increase in phototpic ERG amplitudes in treated eyes compared to controls (Figure 5B). Flicker ERGs confirmed that the recovery mechanism was still active in these cone cells expressing GIRK2 allowing them to follow a fast stimulus (Figure 5C). The rd10 animals treated with GIRK2 showed also an improved optokinetic reflex compared to controls (Figure 5D).

Next it was studied if the endogenous cone opsin, still present in degenerating cones, was functional and sufficient to activate GIRK2 channel in this mouse model. For this, a single AAV8 vector encoding GIRK2 in fusion with GFP was delivered. This led to similar increases in photopic ERG amplitudes and optokinetic reflex in treated eyes compared to controls confirming that GIRK2 alone was sufficient to increase light sensitivity via G protein coupled signalling involving cone opsin (Figure 6A-B). Flicker ERGs were also robustly amplified with this approach (Figure 6C).

### 3. GIRK2-mediated vision restoration: long-term efficacy

Photopic ERG recordings were performed to monitor the cone response to light stimuli at different time points after treatment with GIRK2 and in absence of treatment. These ERGs were done under two conditions: (i) photopic with light flashes applied every second during 60 seconds at increasing light intensities and (ii) flicker stimulation with repetitive flashes during 60 seconds. Data were collected on a weekly basis until p50 and then every 10 to 13 days until 11 weeks of age and showed a gradual decline in ERG amplitudes for both controls and treated eyes (Figure 7A). Moreover, these results are consistent with the optokinetic test, both controls and treated eyes with GIRK2 show a decreased optokinetic reflex over time (Figure 7B). This decline was to be expected as cone numbers also decreased over time in the rd10 mice (Figure 7C). The number of cone photoreceptors remaining in rd10 retinas were counted to correlate decreases in cone numbers with decrease in ERG amplitudes. Indeed, the decrease in light responses was proportional to number of remaining photoreceptors (Figure 7D). It was thus concluded that GIRK2 increased light responses in remaining cones so long as cones remained alive but, as expected, it did not slow down the loss of cone cells.

**4. GIRK2-mediated vision restoration in an RCD model caused by mutant rhodopsin**

Having in mind the goal of creating a mutation-independent therapy, the approach was tested in another mouse model - with a different causal mutation. To this aim experiments were done in a heterozygous mouse model called mRho$^{-/-}$huRhoP347S$^{+/-}$ carrying a knock in for P347S mutant human rhodopsin. Mutant human rhodopsin and absence of mouse rhodopsin led to a rod-cone dystrophy in this complementary model. Here, the same set of experiments was repeated as that was done in the rd10 mouse model. First, the phototransduction cascade proteins interacting with cone opsin at different time points was analysed (Figure 8). It was noticed that: (i) the degeneration rate was slower compared to rd10 and (ii) similar to the rd10 model only the opsin and the arrestin persisted in cone cell bodies at P150.

Next, mice were injected at P15 with the same AAV vectors encoding for GIRK2 fused with GFP and recorded ERGs to monitor cone response to light stimuli at various time points (Figure 9A). The response amplitudes of treated eyes were significantly higher than that of control eyes until P100. Moreover, flicker ERG responses were also similarly improved in this mouse model. Similarly to rd10 mice, this mouse model also shows an improved optokinetic reflex that decreases over time in both control and treated conditions (Figure 9B). This decline is to be expected as cone numbers also decreases over time in this RCD mouse model (Figure 9C). The decrease in time in ERG amplitudes also correlated with a decrease in cone numbers in this model (Figure 9D). This was again consistent with the fact that the approach did not stop the degeneration but allowed for enhanced light sensitivity via GIRK2.

**5. Efficiency of the mouse GIRK2 in an *in vitro* test**

Light stimulations (400nm, 5 seconds, fullfield) activated GIRK currents in HEK cells expressing both GIRK and SWO (Short Wavelength Opsin) (Figure 10). GIRK channels are modulated in a membrane-delimited, fast manner via the Gi/o pathway and the expression of the mouse GIRK channel was membrane bound. The amplitudes and kinetics of light-induced activation and deactivation of GIRK channels with SWO, induce large GIRK current amplitudes during a 5 s light pulse.

**6. GIRK2-mediated vision restoration in RP human patients**

To check the applicability of this GIRK approach at the cellular level in the human retina, post-mortem retinal specimens have been used to analyze phototransduction cascade proteins in the macular region in 4 RP patients aged between 73 and 92 years old who were in intermediate and advanced stages of the disease (Postmortem eyes obtained from the Cole Eye Institute Eye Tissue Repository through the Foundation Fighting Blindness (FFB) Eye Donor Program (Columbia, MD)). The expression of phototransduction cascade proteins have also been analyzed in a healthy control retina from a 91-year-old patient (tissue was obtained from the Surgery School of Paris). Importantly, it has been found that similar to RCD mouse models, cone opsin and cone arrestin remain in cone cell bodies of RP patients (Data not shown). Indeed, three of four donors (Donor 2 to 4) had cones with diminished outer segments. In all 3 patients, cones were found co-expressing opsin and arrestin (Data not shown). The donor 5, who was blind, had an extremely low number of cones (Data not shown). Altogether these data confirm the applicability of GIRK-mediated gene therapy in human cones. The short phototransduction cascade strategy can potentially reactivate cone function in RCD patients. The activation of remaining cone opsin by a light stimulus may trigger the short GIRK2-mediated phototransduction cascade and lead to enhanced light sensitivity in RP patients.

In addition, proportion of patients having the two criteria: (i) visual acuity <2/10 with low to no light perception and (ii) presence of a detectable ONL filled with cone photoreceptor cells displaying shortened or absent outer segments (referred to as 'dormant cones'), have been estimated though the RCD patients. A database of 350 eyes with genetically confirmed Retinitis Pigmentosa (RP) diagnosis has been screened and eyes having a visual acuity below 2/10 (Figure 14A) have been sorted. 115 eyes presented a very reduced or lost light perception (Fig. 14A) were then further sorted out. Then, the presence of a discernible ONL by examining the fine structure of the foveal region containing the cone cells last to degenerate in RCD, have been examined (also referred to in the literature as remnant cones or dysflective cones [61]). Among the 115 eyes with low to no light perception, 29 eyes had a distinguishable outer nuclear layer (ONL) composed of cones with shortened or absent outer-segments (Fig. 14 A-E). This indicates that roughly one quarter of RP patients with low to no light perception can be eligible for GIRK2 therapy. The GIRK2 therapy approach may then allow to increase light sensitivity in degenerating cone photoreceptor cells and allow roughly one quarter of RP patients with low to no light perception to regain light perception and high acuity vision in the fovea.

In order to deeply characterize the dormant cone phenotype, and shed light into the inner segment structures overlying the diminished outer segments, the cone cells of one patient who had a very high-quality OCT scan using adaptive optics scanning laser ophthalmoscopy (AOSLO) (Physical Sciences Inc., Andover MA, USA) have been examined (Fig. 14C). This has revealed intact cones in the confocal channel and intact inner segments in the split detection channel. AOSLO imaging revealed refractive changes in the inner and outer segments of the foveal cones, suggesting that it is possible to identify the presence of light insensitive 'dormant' cone population using this technique (Fig. 14G). The combination of the above-mentioned imaging techniques along with more recently described indicators of cone cell function (such as fundus autofluorescence imaging [62]), makes it feasible to select patient populations that would most benefit from GIRK2 gene therapy.

**List of references**

1. Sinha R, Hoon M, Baudin J, Okawa H, Wong ROL, Rieke F. 2017. Cellular and Circuit Mechanisms Shaping the Perceptual Properties of the Primate Fovea. Cell. 168(3):413-426.e12

2. Yau KW, Hardie RC. 2009. Phototransduction motifs and variations. Cell. 139(2):246-64

3. Ebrey T, Koutalos Y. 2001. Vertebrate photoreceptors. Prog Retin Eye Res. 20(1):49-94

4. Larhammar D, Nordström K, Larsson T a. 2009. Evolution of vertebrate rod and cone phototransduction genes. Philosophical transactions of the Royal Society of London. Series B, Biological sciences. 364(1531):2867-80

5. Maeda T, Imanishi Y, Palczewski K. 2003. Rhodopsin phosphorylation: 30 Years later. Prog. Retin Eye Res. 22(4): 417-434

6. Buch H, Vinding T, La Cour M, Appleyard M, Jensen GB, Nielsen NV. 2004. Prevalence and Causes of Visual Impairment and Blindness among 9980 Scandinavian Adults: The Copenhagen City Eye Study. Ophthalmology. 111(1):53-61

7. Wright AF, Chakarova CF, Abd El-Aziz MM, Bhattacharya SS. 2010. Photoreceptor degeneration: genetic and mechanistic dissection of a complex trait. Nature reviews. Genetics. 11(4):273-84

8. Ferrari S, Di Iorio E, Barbaro V, Ponzin D, Sorrentino FS, Parmeggiani F. 2011. Retinitis pigmentosa: genes and disease mechanisms. Current genomics. 12(4):238-49

9. Li ZY, Kljavin IJ, Milam a H. 1995. Rod photoreceptor neurite sprouting in retinitis pigmentosa. The Journal of neuroscience : the official journal of the Society for Neuroscience. 15(8):5429-38

10. Bennett J. 2017. Taking Stock of Retinal Gene Therapy: Looking Back and Moving Forward. Molecular Therapy. 25(5):1076-94

11. Dalkara D, Duebel J, Sahel J-A. 2015. Gene therapy for the eye focus on mutation-independent approaches. Current Opinion in Neurology. 28(1):51-60

12. Busskamp V, Picaud S, Sahel JA, Roska B. 2012. Optogenetic therapy for retinitis pigmentosa. Gene Therapy. 19(2):169-75

13. Dalkara D, Sahel J-A. 2014. Gene therapy for inherited retinal degenerations. C. R. Biol. 337(3): 185-192

14. Scholl HPN, Strauss RW, Singh MS, Dalkara D, Roska B, et al. 2016. Emerging therapies for inherited retinal degeneration. Science Translational Medicine. 8(368):368rv6-368rv6

15. Baker CK, Flannery JG. 2018. Innovative Optogenetic Strategies for Vision Restoration. Frontiers in Cellular Neuroscience. 12(September):1-8

16. Cehajic-Kapetanovic J, Eleftheriou C, Allen AE, Milosavljevic N, Pienaar A, et al. 2015. Restoration of Vision with Ectopic Expression of Human Rod Opsin. Current Biology. 25(16):2111-22

17. Gaub BM, Berry MH, Holt AE, Isacoff EY, Flannery JG. 2015. Optogenetic Vision Restoration Using Rhodopsin for Enhanced Sensitivity. Molecular therapy: the journal of the American Society of Gene Therapy. 23(10):1562-71

18. Van Gelder RN, Kaur K. 2015. Vision Science: Can Rhodopsin Cure Blindness. Current Biology. 25(16):R713-15

19. van Wyk M, Pielecka-Fortuna J, Löwel S, Kleinlogel S. 2015. Restoring the ON Switch in Blind Retinas: Opto-mGluR6, a Next-Generation, Cell-Tailored Optogenetic Tool. PLOS Biology. 13(5):e1002143

20. Berry MH, Holt A, Levitz J, Visel M, Aghi K, et al. Restoration of high-sensitivity , adapting , patterned vision with a cone opsin. Nature Communications. (2019):1-12

21. De Silva SR, Barnard AR, Hughes S, Tam SKE, Martin C, et al. 2017. Long-term restoration of visual function in end-stage retinal degeneration using subretinal human melanopsin gene therapy. Proceedings of the National Academy of Sciences. 114(42):11211-16

22. Lin B, Koizumi A, Tanaka N, Panda S, Masland RH. 2008. Restoration of visual function in retinal degeneration mice by ectopic expression of melanopsin. Proceedings of the National Academy of Sciences of the United States of America. 105(41):16009-14

23. Masseck OA, Spoida K, Dalkara D, Maejima T, Rubelowski JM, et al. 2014. Vertebrate Cone Opsins Enable Sustained and Highly Sensitive Rapid Control of G i/o Signaling in Anxiety Circuitry. Neuron. 81(6):1263-73

24. Mark MD, Herlitze S. 2000. G-protein mediated gating of inward-rectifier K+ channels. Eur. J. Biochem. 267(19): 5830-5836

25. Busskamp V, Duebel J, Balya D, Fradot M, Viney TJ, et al. 2010. Genetic Reactivation of Cone Photoreceptors Restores Visual Responses in Retinitis Pigmentosa. Science. 329(5990):413-17

26. Packer AM, Roska B, Häusser M. 2013. Targeting neurons and photons for optogenetics. Nature neuroscience. 16(7):805-15

27. Khabou H, Garita-Hernandez M, Chaffiol A, Reichman S, Jaillard C, et al. 2018. Noninvasive gene delivery to foveal cones for vision restoration. JCI Insight. 3(2):1-18

28. International application WO 2018134168

29. Byrne LC, Dalkara D, Luna G, Fisher SK, Clérin E, et al. 2015. Viral-mediated RdCVF and RdCVFL expression protects cone and rod photoreceptors in retinal degeneration. Journal of Clinical Investigation. 125(1):105-16

30. Millington-Ward S, Chadderton N, O'Reilly M, Palfi A, Goldmann T, et al. 2011. Suppression and Replacement Gene Therapy for Autosomal Dominant Disease in a Murine Model of Dominant Retinitis Pigmentosa. Molecular Therapy. 19(4):642-49

31. Ma et al., 2002. Neuron. 33: 715-729

32. International application WO 2012145601

33. Ye et al., 2016. Hum. Gene Ther. 27(1):72-82

34. Khabou et al., 2018. JCI Insight. 3(2):e96029

35. International application WO 2015142941

36. International application WO 2018055131

37. Watanabe K et al.,2005. Nat Neurosci. 8(3):288-96

38. Osakada F et al., 2009. Nat. Protoc. 4(6):811-24

39. Osakada F et al., 2008. Nat Biotechnol. 26(2):215-24

40. Amirpour N et al., 2012. Stem Cells Dev. 21(I):42-53

41. Nakano T et al., 2012. Cell Stem Cell. 10(6):771-85

42. Zhu Y et al., 2013. Plos One. 8(I):e54552

43. Yanai A et al., 2013. Tissue Eng Part C Methods. 19(10):755-64

44. Kuwahara A et al., 2015. Nat Commun. 6:6286

45. Mellough CB et al., 2015. Stem Cells. 33(8):2416-30

46. Singh K et al., 2015. Stem Cells Dev. 24(23):2778-95

47. Garita-Hernandez et al., 2019. Nat. Commun. 10:4524

48. Lamba et al., 2006. Proc Natl Acad Sci USA. 103(34):12769-74

49. Lamba et al., 2010. Plos one. 5(I):e8763

50. Meyer JS et al., 2009. Proc Natl Acad Sci USA. 106(39):16698-703

51. Meyer JS et al., 2011. Stem Cells. 29(8) : 1206-18

52. Mellough CB et al., 2012. Stem Cells. 30(4):673-86

53. Boucherie C et al., 2013. Stem Cells. 31(2) :408-14

54. Sridhar A et al., 2013. Stem Cells Transl Med. 2(4) :255-64

55. Tucker BA et al., 2013. Elife. 2:e00824

56. Tucker BA et al., 2013. Stem Cells Transl Med. 2(1) :16-24

57. Eichman S et al., 2014. Proc Natl Acad Sci USA. 111(23) :8518-23

58. Zhong X et al., 2014. Nat Commun. 5 :4047

59. Wang X et al., 2015. Biomaterials. 53 :40-9

60. Roorda et al., 2002. Opt Exp. 10(9):405-12

61. J. L. Duncan, A. Roorda. Retinal Degenerative Diseases Mechanisms and Experimental Therapy, (2019), pp. 133-137

62. D. L. Greenwald, S. M. Cashman, R. Kumar-Singh. Mutation-independent rescue of a novel mouse model of Retinitis Pigmentosa, Gene Therapy 20, 425-434 (2013)

63. Azzam D, Ronquillo Y. Snellen chart. 2021 May 9. In: StatPearls [Internet]. Treasure Island (FL): StatPearls Publishing

64. David Caltrider, Abhishek Gupta, Koushik. Evaluation Of Visual Acuity. In: StatPearls [Internet]. Treasure Island (FL): StatPearls Publishing; 2021 Jan. 2021 Feb 14

65. Davies et al., Retinal Ganglion Cell Layer Volumetric Assessement by Spectral-domain OCT in MS: Application of a High Precision Manual Estimation Technique. J Neuroophthalmol. 2011; 31(3):260-264

66. Podoleanu, Optical coherence tomography. Journal of Microscopy, 2012

67. Iorga Raluca Eugenia et al., The role of optical coherence tomography in optic neuropathies. Romanian Journal of Ophthalmology, Volume 62, Issue 1, January-March 2018; pp:3-14

68. Chen Y. et al., Diabetic macular morphology changes may occur in the early stage of diabetes. BMC Ophthalmology (2016) 16:12

69. Horton et al., Spontaneous regeneration of human photoreceptor outer segments. Nature Scientific reports, 2015

70. Merino et al., Adaptative optics scanning laser ophthalmoscope imaging: technology update. Clinical Ophthalmology 2016:10, 743-755

**Claims**

1. A vector comprising a nucleotide sequence encoding subunit 2 of G-protein-gated inwardly rectifying potassium channel (GIRK2) or a functional derivative thereof, or a carrier comprising said vector for use in the treatment of rod-cone dystrophy (RCD) in a subject.

2. The vector or the carrier for use according to claim 1, wherein said subject has a presence of cone photoreceptor cells displaying shortened or absent outer-segments in the outer nuclear layer (ONL).

3. The vector or the carrier for use according to claim 1 or 2, wherein said subject has a visual acuity equal or inferior to 6/10, in particular equal or inferior to 5/10, 4/10, 3/10 or 2/10.

4. The vector or the carrier comprising said vector for use according to any of claims 1 to 3, wherein the nucleotide sequence encoding GIRK2 or a derivative thereof comprises the sequence SEQ ID NO:1, SEQ ID NO:3 or SEQ ID NO:5.

5. The vector or the carrier comprising said vector for use according to any one of claim 1 to 4, wherein the vector is selected from the group consisting of an adeno-associated virus (AAV), an adenovirus, a lentivirus, and SV40 viral vector.

6. The vector or the carrier comprising said vector for use according to any one of claims 1 to 5, wherein the vector is an AAV2 or AW9 virus comprising a 7 to 11 amino acid long insertion peptide in the GH loop of the VP1 capsid protein, wherein the insertion peptide comprises amino acid sequence LGETTRP (SEQ ID NO: 7).

7. The vector or the carrier comprising said vector for use according to any one of claims 1 to 6, wherein the vector is a recombinant AAV9 vector comprising:

   - a VP1 capsid protein in a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
   - the nucleotide sequence encoding GIRK2 or a functional derivative thereof under the control of a pR1.7 promoter.

8. The vector or the carrier comprising said vector for use according to claim 6 or 7, wherein said insertion peptide comprises or consists of amino acid sequence AALGETTRPA (SEQ ID NO: 10), LALGETTRPA (SEQ ID NO: 11), or GLGETTRPA (SEQ ID NO: 12).

9. The vector or the carrier comprising said vector for use according to any one of claims 1 to 8, wherein said vector further comprises a nucleotide sequence encoding a mammalian cone opsin.

10. The carrier comprising the vector for use according to any one of claims 1 to 8, wherein said carrier further includes a vector comprising a nucleotide sequence encoding a mammalian cone opsin.

11. The carrier comprising the vector for use according to claim 10, wherein the vector comprising a nucleotide sequence encoding a mammalian cone opsin:

    a) is selected from the group consisting of an adeno-associated virus (AAV), an adenovirus, a lentivirus, and SV40 viral vector; or
    b) is an AAV2 or AW9 virus comprising a 7 to 11 amino acid long insertion peptide in the GH loop of the VP1 capsid protein, wherein the insertion peptide comprises amino acid sequence LGETTRP (SEQ ID NO: 7);or
    c) is a recombinant AAV9 vector comprising:

       - a VP1 capsid protein in a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
       - the nucleotide sequence encoding the mammalian cone opsin under the control of a pR1.7 promoter.

12. The carrier comprising the vector for use according to any one of claims 1 to 11, wherein the carrier is chosen from the group consisting of solid-lipid nanoparticles, chitosan nanoparticles, liposome, lipoplex and cationic polymer.

13. The vector for use according to claim 8 or carrier for use according to any one of claims 10 to 12, wherein the mammalian cone opsin is a short wavelength cone opsin (SWO).

14. The vector or the carrier comprising said vector, for use according to any one of claims 1 to 13, wherein said vector or said carrier is comprised in a pharmaceutical composition along with a pharmaceutically acceptable carrier, diluent or excipient.

15. The vector or the carrier comprising said vector for use according to any one of claims 1 to 14, wherein the vector, carrier or pharmaceutical composition is administered by subretinal injection at distance of the fovea.

16. The vector or the carrier comprising said vector for use according to claim 15, wherein the vector, carrier or pharmaceutical composition is administered by subretinal injection a) in a region adjacent to the superior or inferior temporal branch of retinal artery; b) at a distance of 2-3 optic disk diameter away from the center of the fovea; and c) at a position localized in the geometric shape delineated by the branches of temporal retinal artery and temporal retinal vein.

A

B

FIGURE 1

A

EP 4 163 296 A1

| Score | | Expect | Method | Identities | Positives | Gaps |
|---|---|---|---|---|---|---|
| 845 bits(2182) | | 0.0 | Compositional matrix adjust. | 404/407(99%) | 407/407(100%) | 0/407(0%) |

**mouse**    Query   19    MDQDVESPVAIHQPKLPKQARDDLPRHISRDRTKRKIQRYVRKDGKCNVHHGNVRETYRY    78
**rat**     Sbjct    1     ...........................................R...................    60

Query   79    LTDIFTTLVDLKWRFNLLIFVMVYTVTWLFFGMIWWLIAYIRGDMDHIEDPSWTPCVTNL    138
Sbjct   61    ............................................................    120

Query   139    NGFVSAFLFSIETETTIGYGYRVITDKCPEGIILLLIQSVLGSIVNAFMVGCMFVKISQP    198
Sbjct   121    ............................................................    180

Query   199    KKRAETLVFSTHAVISMRDGKLCLMFRVGDLRNSHIVEASIRAKLIKSKQTSEGEFIPLN    258
Sbjct   181    ............................................................    240

Query   259    QTDINVGYYTGDDRLFLVSPLIISHEINQQSPFWEISKAQLPKEELEIVVILEGMVEATG    318
Sbjct   241    ............................................................    300

Query   319    MTCQARSSYITSEILWGYRFTPVLTLEDGFYEVDYNSFHETYETSTPSLSAKELAELANR    378
Sbjct   301    ...........V............................H...................    360

Query   379    AELPLSWSVSSKLNQHAELETEEEEKNPEELTERNGDVANLENESKV    425
Sbjct   361    ...............................................    407

3 AA difference in mouse variant: R54K, V328I, H360Y

FIGURE 2

B

| Score | Expect | Method | Identities | Positives | Gaps |
|---|---|---|---|---|---|
| 870 bits(2247) | 0.0 | Compositional matrix adjust. | 418/423(99%) | 419/423(99%) | 0/423(0%) |

```
        Query    3   MAKLTESMTNAAEGDSMDQDVESPVAIHQPKLPKQARDDLPRHISRDRTKRKIQRYVRKD   62
human   Sbjct    1   ................VL..........................................   60

        Query   63   GKCNVHHGNVRETYRYLTDIFTTLVDLKWRFNLLIFVMVYTVTWLFFGMIWWLIAYIRGD   122
        Sbjct   61   ...........................................................   120

        Query  123   MDHIEDPSWTPCVTNLNGFVSAFLFSIETETTIGYGYRVITDKCPEGIILLLIQSVLGSI   182
        Sbjct  121   ...........................................................   180

        Query  183   VNAFMVGCMFVKISQPKKRAETLVFSTHAVISMRDGKLCLMFRVGDLRNSHIVEASIRAK   242
        Sbjct  181   ...........................................................   240

        Query  243   LIKSKQTSEGEFIPLNQTDINVGYYTGDDRLFLVSPLIISHEINQQSPFWEISKAQLPKE   302
        Sbjct  241   ...........................................................   300

        Query  303   ELEIVVILEGMVEATGMTCQARSSYITSEILWGYRFTPVLTLEDGFYEVDYNSFHETYET   362
        Sbjct  301   ...........................................................   360

        Query  363   STPSLSAKELAELANRAELPLSWSVSSKLNQHAELETEEEEKNPEELTERNGDVANLENE   422
        Sbjct  361   .................S..............................L..Q........   420

        Query  423   SKV       425
        Sbjct  421   ...       423
```

3 AA difference in human variant (not counting VL to AA) : N375S, P404L, L407Q

FIGURE 2 (FIN)

FIGURE 3

B

452_pAAV-CMV-mSWO-mCherry originale
6544 bp

FIGURE 3 (FIN)

FIGURE 4

FIGURE 5

**A** Photopic ERG at P41

**B** Optokinetic reflex at P41

**C**

WT

GFP

SWO+GIRK2

GIRK2

FIGURE 6

FIGURE 7

EP 4 163 296 A1

FIGURE 8

A: WT, Opsin
B: WT, Transducin
C: WT, PDE
D: WT, Cone arrestin
E: huP347S+/- P14, Opsin
F: huP347S+/- P14, Transducin
G: huP347S+/- P14, PDE
H: huP347S+/- P14, Cone arrestin
I: huP347S+/- P150, Opsin
J: huP347S+/- P150, Transducin
K: huP347S+/- P150, PDE
L: huP347S+/- P150, Cone arrestin

FIGURE 9

FIGURE 10

FIGURE 11

FIGURE 12

FIGURE 13

FIGURE 14

Europäisches
Patentamt

European
Patent Office

Office européen
des brevets

## EUROPEAN SEARCH REPORT

**Application Number**

EP 22 20 0939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Institute For Ophthalmic Research Tuebingen: ""Retinal Gene Therapy for Vision Restoration" – Dr. Deniz Dalkara", , 26 February 2021 (2021-02-26), XP055901712, Retrieved from the Internet: URL:https://www.youtube.com/watch?v=z4ORuqEv5XY | 1-5,12, 14,15 | INV. C07K14/705 A61P27/02 A61K38/17 A61K48/00 |
| Y | * minutes 19:48 – 24:22 * | 6-8,16 | |
| X,P | WO 2021/204407 A1 (CENT NAT RECH SCI) 14 October 2021 (2021-10-14) * the whole document * * examples 1,2 * * page 7, lines 20-24 * * page 9, lines 1-10 * * sequences 1-16 * | 1-5,9, 10,13-15 | |
| X,P | EP 3 892 738 A1 (UNIV SORBONNE [FR]; CENTRE NAT RECH SCIENT [FR] ET AL.) 13 October 2021 (2021-10-13) * the whole document * * examples 1,2 * * paragraph [0017]; sequences 1-6 * | 1-5,9, 10,13-15 | TECHNICAL FIELDS SEARCHED (IPC)  C07K A61P A61K C12N A01K |
| Y,D | HANEN KHABOU ET AL: "Noninvasive gene delivery to foveal cones for vision restoration", JCI INSIGHT, vol. 3, no. 2, 25 January 2018 (2018-01-25), XP055454489, DOI: 10.1172/jci.insight.96029 * abstract * | 6-8,16 | |

-/--

The present search report has been drawn up for all claims

| Place of search | Date of completion of the search | Examiner |
|---|---|---|
| Munich | 3 March 2023 | Sommer, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 22 20 0939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Simon Cardillia-Joe ET AL: "Reactivating the phototransduction cascade by universally applicable gene therapy preserves retinal function in Rod-Cone dystrophy-Vingts National Eye Hospital Jens Duebel Michel paques Quinze-Vingts hospital", , 17 January 2022 (2022-01-17), pages 1-24, XP055900996, Retrieved from the Internet: URL:https://assets.researchsquare.com/file s/rs-1189099/v1/3f56f492-9853-47b5-9694-da f428f59c12.pdf?c=1644247619 [retrieved on 2022-03-14] * the whole document * ----- | | |
| A,D | MASSECK OLIVIA A ET AL: "Vertebrate Cone Opsins Enable Sustained and Highly Sensitive Rapid Control of Gi/oSignaling in Anxiety Circuitry", NEURON, vol. 81, no. 6, 19 March 2014 (2014-03-19) , pages 1263-1273, XP028656615, ISSN: 0896-6273, DOI: 10.1016/J.NEURON.2014.01.041 * the whole document * ----- | 1-16 | TECHNICAL FIELDS SEARCHED (IPC) |

-/--

The present search report has been drawn up for all claims

| Place of search | Date of completion of the search | Examiner |
|---|---|---|
| Munich | 3 March 2023 | Sommer, Birgit |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BERRY MICHAEL H. ET AL: "Restoration of high-sensitivity and adapting vision with a cone opsin", NATURE COMMUNICATIONS , vol. 10, no. 1 1 December 2019 (2019-12-01), XP055858939, DOI: 10.1038/s41467-019-09124-x Retrieved from the Internet: URL:https://www.ncbi.nlm.nih.gov/pmc/artic les/PMC6420663/pdf/41467_2019_Article_9124 .pdf * the whole document * ----- | 1-16 | |

TECHNICAL FIELDS
SEARCHED       (IPC)

The present search report has been drawn up for all claims

| Place of search | Date of completion of the search | Examiner |
|---|---|---|
| Munich | 3 March 2023 | Sommer, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

# ANNEX TO THE EUROPEAN SEARCH REPORT
## ON EUROPEAN PATENT APPLICATION NO.

EP 22 20 0939

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2023

| Patent document cited in search report | | Publication date | Patent family member(s) | | Publication date |
|---|---|---|---|---|---|
| WO 2021204407 | A1 | 14-10-2021 | EP | 4132961 A1 | 15-02-2023 |
| | | | WO | 2021204407 A1 | 14-10-2021 |
| EP 3892738 | A1 | 13-10-2021 | NONE | | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018134168 A
- WO 2012145601 A
- WO 2015142941 A
- WO 2018055131 A


**Non-patent literature cited in the description**

- *Hum Gene Ther.,* January 2016, vol. 27 (1), 72-82
- **SINHA R ; HOON M ; BAUDIN J ; OKAWA H ; WONG ROL ; RIEKE F.** Cellular and Circuit Mechanisms Shaping the Perceptual Properties of the Primate Fovea. *Cell,* 2017, vol. 168 (3), 413-426.e12
- **YAU KW ; HARDIE RC.** Phototransduction motifs and variations. *Cell,* 2009, vol. 139 (2), 246-64
- **EBREY T ; KOUTALOS Y.** Vertebrate photoreceptors. *Prog Retin Eye Res.,* 2001, vol. 20 (1), 49-94
- **LARHAMMAR D ; NORDSTRÖM K ; LARSSON T A.** Evolution of vertebrate rod and cone phototransduction genes. *Philosophical transactions of the Royal Society of London. Series B, Biological sciences,* 2009, vol. 364 (1531), 2867-80
- **MAEDA T ; IMANISHI Y ; PALCZEWSKI K.** Rhodopsin phosphorylation: 30 Years late. *Prog. Retin Eye Res.,* 2003, vol. 22 (4), 417-434
- **BUCH H ; VINDING T ; LA COUR M ; APPLEYARD M ; JENSEN GB ; NIELSEN NV.** Prevalence and Causes of Visual Impairment and Blindness among 9980 Scandinavian Adults: The Copenhagen City Eye Study. *Ophthalmology,* 2004, vol. 111 (1), 53-61
- **WRIGHT AF ; CHAKAROVA CF ; ABD EL-AZIZ MM ; BHATTACHARYA SS.** Photoreceptor degeneration: genetic and mechanistic dissection of a complex trait. *Nature reviews. Genetics.,* 2010, vol. 11 (4), 273-84
- **FERRARI S ; DI IORIO E ; BARBARO V ; PONZIN D ; SORRENTINO FS ; PARMEGGIANI F.** Retinitis pigmentosa: genes and disease mechanisms. *Current genomics,* 2011, vol. 12 (4), 238-49
- **LI ZY ; KLJAVIN IJ ; MILAM A H.** Rod photoreceptor neurite sprouting in retinitis pigmentosa. *The Journal of neuroscience : the official journal of the Society for Neuroscience.,* 1995, vol. 15 (8), 5429-38
- **BENNETT J.** Taking Stock of Retinal Gene Therapy: Looking Back and Moving Forward. *Molecular Therapy.,* 2017, vol. 25 (5), 1076-94
- **DALKARA D ; DUEBEL J ; SAHEL J-A.** Gene therapy for the eye focus on mutation-independent approaches. *Current Opinion in Neurology.,* 2015, vol. 28 (1), 51-60
- **BUSSKAMP V ; PICAUD S ; SAHEL JA ; ROSKA B.** Optogenetic therapy for retinitis pigmentosa. *Gene Therapy.,* 2012, vol. 19 (2), 169-75
- **DALKARA D ; SAHEL J-A.** Gene therapy for inherited retinal degenerations. *C. R. Biol.,* 2014, vol. 337 (3), 185-192
- **SCHOLL HPN ; STRAUSS RW ; SINGH MS ; DALKARA D ; ROSKA B et al.** Emerging therapies for inherited retinal degeneration. *Science Translational Medicine.,* 2016, vol. 8 (368), 368rv6-368rv6
- **BAKER CK ; FLANNERY JG.** Optogenetic Strategies for Vision Restoration. *Frontiers in Cellular Neuroscience.,* 2018, vol. 12, 1-8
- **CEHAJIC-KAPETANOVIC J ; ELEFTHERIOU C ; ALLEN AE ; MILOSAVLJEVIC N ; PIENAAR A et al.** Restoration of Vision with Ectopic Expression of Human Rod Opsin. *Current Biology.,* 2015, vol. 25 (16), 2111-22
- **GAUB BM ; BERRY MH ; HOLT AE ; ISACOFF EY ; FLANNERY JG.** Optogenetic Vision Restoration Using Rhodopsin for Enhanced Sensitivity. *Molecular therapy: the journal of the American Society of Gene Therapy.,* 2015, vol. 23 (10), 1562-71
- **VAN GELDER RN ; KAUR K.** Vision Science: Can Rhodopsin Cure Blindness. *Current Biology,* 2015, vol. 25 (16), R713-15
- **VAN WYK M ; PIELECKA-FORTUNA J ; LÖWEL S ; KLEINLOGEL S.** Restoring the ON Switch in Blind Retinas: Opto-mGluR6, a Next-Generation, Cell-Tailored Optogenetic Tool. *PLOS Biology,* 2015, vol. 13 (5), e1002143
- **BERRY MH ; HOLT A ; LEVITZ J ; VISEL M ; AGHI K et al.** Restoration of high-sensitivity , adapting , patterned vision with a cone opsin. *Nature Communications,* 2019, 1-12

- **DE SILVA SR ; BARNARD AR ; HUGHES S ; TAMSKE ; MARTIN C et al.** Long-term restoration of visual function in end-stage retinal degeneration using subretinal human melanopsin gene therapy. *Proceedings of the National Academy of Sciences,* 2017, vol. 114 (42), 11211-16
- **LIN B ; KOIZUMI A ; TANAKA N ; PANDA S ; MASLAND RH.** Restoration of visual function in retinal degeneration mice by ectopic expression of melanopsin. *Proceedings of the National Academy of Sciences of the United States of America,* 2008, vol. 105 (41), 16009-14
- **MASSECK OA ; SPOIDA K ; DALKARA D ; MAEJIMA T ; RUBELOWSKI JM et al.** Vertebrate Cone Opsins Enable Sustained and Highly Sensitive Rapid Control of G i/o Signaling in Anxiety Circuitry. *Neuron.,* 2014, vol. 81 (6), 1263-73
- **MARK MD ; HERLITZE S.** G-protein mediated gating of inward-rectifier K+ channels. *Eur. J. Biochem.,* 2000, vol. 267 (19), 5830-5836
- **BUSSKAMP V ; DUEBEL J ; BALYA D ; FRADOT M ; VINEY TJ et al.** Genetic Reactivation of Cone Photoreceptors Restores Visual Responses in Retinitis Pigmentosa. *Science,* 2010, vol. 329 (5990), 413-17
- **PACKER AM ; ROSKA B ; HÄUSSER M.** Targeting neurons and photons for optogenetics. *Nature neuroscience.,* 2013, vol. 16 (7), 805-15
- **KHABOU H ; GARITA-HERNANDEZ M ; CHAFFIOL A ; REICHMAN S ; JAILLARD C et al.** Noninvasive gene delivery to foveal cones for vision restoration. *JCI Insight.,* 2018, vol. 3 (2), 1-18
- **BYRNE LC ; DALKARA D ; LUNA G ; FISHER SK ; CLÉRIN E et al.** Viral-mediated RdCVF and RdCVFL expression protects cone and rod photoreceptors in retinal degeneration. *Journal of Clinical Investigation.,* 2015, vol. 125 (1), 105-16
- **MILLINGTON-WARD S ; CHADDERTON N ; O'REILLY M ; PALFI A ; GOLDMANN T et al.** Suppression and Replacement Gene Therapy for Autosomal Dominant Disease in a Murine Model of Dominant Retinitis Pigmentosa. *Molecular Therapy,* 2011, vol. 19 (4), 642-49
- **MA et al.** *Neuron,* 2002, vol. 33, 715-729
- **YE et al.** *Hum. Gene Ther.,* 2016, vol. 27 (1), 72-82
- **KHABOU et al.** *JCI Insight.,* 2018, vol. 3 (2), e96029
- **WATANABE K et al.** *Nat Neurosci.,* 2005, vol. 8 (3), 288-96
- **OSAKADA F et al.** *Nat. Protoc.,* 2009, vol. 4 (6), 811-24
- **OSAKADA F et al.** *Nat Biotechnol.,* 2008, vol. 26 (2), 215-24
- **AMIRPOUR N et al.** *Stem Cells Dev.,* 2012, vol. 21 (I), 42-53
- **NAKANO T et al.** *Cell Stem Cell,* 2012, vol. 10 (6), 771-85
- **ZHU Y et al.** *Plos One.,* 2013, vol. 8 (I), e54552
- **YANAI A et al.** *Tissue Eng Part C Methods.,* 2013, vol. 19 (10), 755-64
- **KUWAHARA A et al.** *Nat Commun.,* 2015, vol. 6, 6286
- **MELLOUGH CB et al.** *Stem Cells.,* 2015, vol. 33 (8), 2416-30
- **SINGH K et al.** *Stem Cells Dev.,* 2015, vol. 24 (23), 2778-95
- **GARITA-HERNANDEZ et al.** *Nat. Commun.,* 2019, vol. 10, 4524
- **LAMBA et al.** *Proc Natl Acad Sci USA.,* 2006, vol. 103 (34), 12769-74
- **LAMBA et al.** *Plos one,* 2010, vol. 5 (I), e8763
- **MEYER JS et al.** *Proc Natl Acad Sci USA.,* 2009, vol. 106 (39), 16698-703
- **MEYER JS et al.** *Stem Cells.,* 2011, vol. 29 (8), 1206-18
- **MELLOUGH CB et al.** *Stem Cells.,* 2012, vol. 30 (4), 673-86
- **BOUCHERIE C et al.** *Stem Cells.,* 2013, vol. 31 (2), 408-14
- **SRIDHAR A et al.** *Stem Cells Transl Med.,* 2013, vol. 2 (4), 255-64
- **TUCKER BA et al.** *Elife,* 2013, vol. 2, e00824
- **TUCKER BA et al.** *Stem Cells Transl Med.,* 2013, vol. 2 (1), 16-24
- **EICHMAN S et al.** *Proc Natl Acad Sci USA.,* 2014, vol. 111 (23), 8518-23
- **ZHONG X et al.** *Nat Commun.,* 2014, vol. 5, 4047
- **WANG X et al.** *Biomaterials,* 2015, vol. 53, 40-9
- **ROORDA et al.** *Opt Exp.,* 2002, vol. 10 (9), 405-12
- **J. L. DUNCAN ; A. ROORDA.** *Retinal Degenerative Diseases Mechanisms and Experimental Therapy,* 2019, 133-137
- **D. L. GREENWALD ; S. M. CASHMAN ; R. KUMAR-SINGH.** Mutation-independent rescue of a novel mouse model of Retinitis Pigmentosa. *Gene Therapy,* 2013, vol. 20, 425-434
- **AZZAM D ; RONQUILLO Y.** Snellen chart. StatPearls Publishing, 09 May 2021
- **DAVID CALTRIDER ; ABHISHEK GUPTA ; KOUSHIK.** Evaluation Of Visual Acuity. StatPearls Publishing, January 2021
- **DAVIES et al.** Retinal Ganglion Cell Layer Volumetric Assessement by Spectral-domain OCT in MS: Application of a High Precision Manual Estimation Technique. *J Neuroophthalmol.,* 2011, vol. 31 (3), 260-264
- **PODOLEANU.** Optical coherence tomography. *Journal of Microscopy,* 2012
- **IORGA RALUCA EUGENIA et al.** The role of optical coherence tomography in optic neuropathies. *Romanian Journal of Ophthalmology,* January 2019, vol. 62 (1), 3-14

- **CHEN Y. et al.** Diabetic macular morphology changes may occur in the early stage of diabetes. *BMC Ophthalmology,* 2016, vol. 16, 12
- **HORTON et al.** Spontaneous regeneration of human photoreceptor outer segments. *Nature Scientific reports,* 2015
- **MERINO et al.** Adaptative optics scanning laser ophthalmoscope imaging: technology update. *Clinical Ophthalmology,* 2016, vol. 10, 743-755